# EUROPEAN PATENT SPECIFICATION

(11) **EP 4 291 273 B1**
(45) Date of publication and mention of the grant of the patent: **17.09.2025**
(21) Application number: 22706557.0
(22) Date of filing: 11.02.2022
(51) Int. Cl.: A61M 15/00, A61K 9/00

(54) **INHALER SYSTEM**
INHALATORSYSTEM
SYSTÈME D'INHALATEUR

(30) Priority: 13.02.2021 US 202163149250 P
(43) Date of publication of application: 20.12.2023
(73) Proprietor: Norton (Waterford) Limited, Waterford (IE)
(72) Inventor: BUCK, Daniel, Waterford (IE); CALDERON OLIVERAS, Enrique, 08172 Barcelona (ES); YANG, Dong, Waterford, X91 C8P7 (IE)
(74) Representative: Elkington and Fife LLP
(86) International application number: PCT/EP2022/053355
(87) International publication number: WO 2022/171791

(56) References cited:
- WO-A1-2014/004437
- WO-A1-2021/022118
- GB-A- 2 558 573
- US-A1- 2011 041 845
- US-A1- 2016 144 141
- US-A1- 2017 290 527
- US-A1- 2019 224 426
- US-A1- 2019 385 727

## Description

### CROSS-REFERENCE TO RELATED APPLICATIONS

This application claims the benefit of Provisional U.S. Patent Application No. 63/149,250, filed February 13, 2021.

### BACKGROUND

Drug delivery devices facilitate the delivery of medication into a patient's body via various routes of administration. Typical routes of administration include oral, topical, sublingual inhalation, injection, and the like. The devices may be used to deliver medications for the treatment various diseases, ailments, and medical conditions. Inhalers, for example, may be used to treat asthma, chronic obstructive pulmonary disease (COPD) and/or cystic fibrosis (CF). While drug delivery devices are designed to deliver an appropriate dose of medication to a patient as part of a therapeutic treatment, the effectiveness of a particular treatment may be influenced by non-physiological factors, such as the patient's adherence and compliance.

In the context of a drug therapy, adherence may refer to the degree to which a patient is following a prescribed dosing regimen. For example, if the patient's prescription calls for two doses each day, and the patient is taking two doses per day, the patient may be considered 100% adherent. If the patient is only taking one dose per day, he or she may be deemed only 50% adherent. In the latter case, the patient may not be receiving the treatment prescribed by his or her doctor, which may negatively affect the efficacy of the therapeutic treatment.

Compliance may refer to a patient's technique when using a particular drug delivery device. If the patient is using the device in a manner that is recommended by a doctor or by a manufacturer, the device is likely to deliver the desired dose of medication and the patient may be deemed compliant. However, if the device is not being used properly during drug administration, the device's ability to deliver a proper dose of medication may be compromised. As such, the patient may be deemed non-compliant. In the case of an inhalation device or inhaler, for example, the patient may need to achieve a minimum inspiratory effort to ensure a full dose of medication is delivered from the device into the patient's lungs. For some patients, such as children and the elderly, meeting the requirements for full compliance may be difficult, for example, due to physical limitations, such as limited lung function. Accordingly, like adherence, failing to achieve full compliance may reduce the effectiveness of a prescribed treatment.

A patient's ability to achieve full compliance may be further complicated by certain physical properties of the medication. For example, some respiratory medications may consist of fine particles and/or may lack any odor or taste. Thus, a patient using an inhaler may not be able to correct a non-compliant use because he or she may not be able to immediately detect or sense that medication is being inhaled and/or know whether the amount of inhaled medication complies with the prescription.

Further, many respiratory diseases, such as asthma and COPD, are life-long conditions where treatment involves the long-term administration of medicaments to manage the patients' symptoms and to decrease the risks of irreversible changes. There is currently no cure for diseases like asthma and COPD. Treatment takes two forms. First, a maintenance aspect of the treatment is intended to reduce airway inflammation and, consequently, control symptoms in the future. The maintenance therapy is typically provided by inhaled corticosteroids, alone or in combination with long-acting bronchodilators and/or muscarinic antagonists. Secondly, there is also a rescue (or reliever) aspect of the therapy, where patients are given rapid-acting bronchodilators to relieve acute episodes of wheezing, coughing, chest tightness and shortness of breath.

Sufferers of respiratory diseases may thus be prescribed more than one medication, such as more than one inhaled medication, for controlling their symptoms. The sufferer may alternatively or additionally make use of a plurality of inhalers, each being used at different times/locations, which all deliver the same inhaled medicament. There is a growing desire to monitor administration of such medicaments in ways which are reliable, and convenient from the point of view of the sufferer. Further, such monitoring is also desirable for the patient's health care provider, who requires a simple and cohesive manner to assess each patient's adherence and compliance, and view data that indicates the highest risk of exacerbation or indicates that the patient's treatment regimen should be altered.

Systems may be designed to monitor a patient's adherence to a prescribed dosing regimen. For example, systems may be designed to monitor a patient's adherence by detecting administrations of a medicament by the patient and confirm that the patient is conforming to the prescribed dosing regimen. In order to detect administrations of the medicament, certain components may be added to or included in the drug delivery device used to administer the medicament. For example, components may be added to detect events or actuations at the drug delivery device that are indicative of an administration of the medicament by the drug delivery device. In addition, communication components may be added to the drug delivery device so that certain information associated with a respective administration of medicament by the drug delivery device (*e.g.,* time of administration, number of remaining doses, *etc*.) can be transmitted to and subsequently stored at and/or analyzed by one or more external devices. Often, these components are add-on components, such as after-market processing and/or communication components that can be attached to a drug delivery device.

However, while these systems may be used to detect and monitor administrations of a drug delivery device and, by extension, measure a patient's adherence to (*e.g.,* the degree to which a patient is following) a prescribed dosing regimen, they fail to measure or monitor the patient's compliance (*e.g.,* the patient's technique when using a particular drug delivery device). For example, while these systems may detect and monitor administrations of medicament by the drug delivery device, they fail to measure the inhalation parameters (*e.g.,* PIF, volume, *etc*.) associated with each administration event. That is, although these systems may be capable of monitoring a patient's adherence to a prescribed dosing regimen, they fail to measure inhalation parameters and thus lack any insight into a patient's compliance. In failing to measure inhalation parameters, these systems fail to appreciate the effectiveness of each inhalation.

The following documents are disclosing inhalers with pressure sensors, namely US 2016/144141 A1, US 2019/224426 A1, WO 2021/022118 A1, US 2017/290527 A1, US 2019/385727 A1, GB 2 558 573 A, WO 2014/004437 A1, US 2011/041845 A1.

### SUMMARY

The invention is defined in the appended claims. An inhaler may include a housing that comprises a mouthpiece, a medication canister, and an airflow channel formed between an air inlet and the mouthpiece. The inhaler may also include a sensor configured to measure a parameter indicative of air flow through the airflow channel of the inhaler. The sensor may be a pressure sensor configured to measure pressure, and/or an acoustic sensor configured to measure acoustic sound waves. Although primarily described as including a pressure sensor, the inhaler described herein may be similarly configured with an acoustic sensor or a flow sensor. The pressure sensor may be a differential pressure sensor or a barometric pressure sensor. The pressure sensor may be attached to the medicament canister of the inhaler. The pressure sensor may be configured to be in a first position relative to the airflow channel when the medication canister is not actuated, and in a second position relative to the airflow channel when the medication canister is in an actuated position. Accordingly, for a predetermined airflow in the airflow channel, the pressure sensor may be configured to measure a first pressure when it is in the first position, and measure a distinguishable second pressure when it is in the second position. The medication canister may be configured to be in a first position when the medication canister is not actuated, and be in a second position when the medication canister is actuated to dispense medication. In some examples, the processor may be configured with a first flow rate calibration curve for when the pressure sensor is in the first position, and a second flow rate calibration curve for when the pressure sensor is in the second position. However, in other examples, the processor and pressure sensor may be calibrated based on only one of the first position or the second position (*e.g.,* configured with only one of the first or second calibration curves). Further, in some examples, the inhaler may include an acoustic sensor instead of a pressure sensor.

Further, in some examples, the housing may also include a hollow socket that is configured to receive a valve stem of the medication canister. In such examples, the pressure sensor may be configured move relative to the hollow socket in response to the medication canister moving to dispense medication.

The inhaler may also include a processor. The processor may be configured to receive pressure measurements from the pressure sensor, and to determine the position of the pressure sensor or the medication canister based on the pressure measurements received from the pressure sensor while the user is inhalation through the mouthpiece of the inhaler. Further, the processor may be configured to determine (*e.g.,* detect) whether an inhalation occurs prior the medication canister being depressed to release medication based on the pressure measurements. The processor may be configured to determine one or more flow rates based on the pressure measurements. As such, the thresholds described herein may be absolute value or slope pressure thresholds or absolute value or slope flow rate thresholds.

The processor may be configured to detect an inhalation event based on the pressure measurements received from the pressure sensor exceeding an inhalation threshold. The processor may be configured to detect a canister actuation event based on the pressure measurements exceeding a canister actuation threshold. The canister actuation threshold may be a slope threshold. The canister actuation threshold may be a larger threshold (*e.g.,* larger, negative threshold) than the inhalation threshold. The processor may be configured to generate an error event based on a slope of the received pressure measurements not exceed a slope threshold that is indicative of the canister actuation during an inhalation. The processor may be configured to generate a late inhalation error event based on the detection of an inhalation that occurs after the medication canister is depressed. The processor may be configured to generate a late actuation error event (*e.g.,* an early inhalation error event) based on the pressure measurements exceeding a threshold for a period of time that exceeds a duration threshold prior to the detection of the canister being actuated. For example, the processor may be configured to determine a peak inspiratory flow (PIF) based on the received pressure measurements, and generate a late actuation error event based on the PIF exceeding a threshold prior to the detection of the canister being actuated. The processor may be configured to generate a good inhalation event based on a slope of the received pressure measurements exceeding a slope threshold that is indicative of the canister actuation during an inhalation. The processor may be configured to generate an on-time inhalation event if the user's inhalation occurred within a predetermined time period before the canister was actuated (*e.g.,* within 300-800 milliseconds before canister actuation).

The processor may be configured to generate one or more parameters (*e.g.,* flow rates, PIF, inhalation volume, inhalation duration, *etc*.) based on the pressure measurements. For example, the processor may be configured to detect a duration of an inhalation event based on the pressure measurements received from the pressure sensor. The duration of the inhalation event may start at the time of the pressure measurements exceed an inhalation threshold (*e.g.,* a slope that is indicative of an inhalation) and end when the parameters return to a predetermined value (*e.g.,* a value indicative of the end of the user's inhalation, such as back to 0 LPM or the atmospheric pressure). Alternatively, the duration of the inhalation event may start at the time of the canister actuation event and end when the parameters return to a predetermined value. The processor is configured to detect the movement of the canister back to the first position upon the release of the canister based on a slope in the pressure measurements exceeding a canister released threshold (*e.g.,* based on a positive change in the slope of the pressure measurements exceeding the canister release threshold).

The inhaler may notify the user of one or more events and/or parameters. For example, the inhaler may include an indicator (*e.g.,* a speaker, display, light, etc.) that is activated to provide feedback to the user based on the detected event type. Alternatively or additionally, the inhaler may also include a transmitter configured to send data to an external device for display via a user interface of the external device. The data may include any combination of the pressure measurements, events (*e.g.,* an inhalation event, a canister actuation event, a late, early, and on-time canister actuation events, *etc*.), and/or parameters that are determined based on the pressure measurements (*e.g.,* flow rates, PIF, inhalation volume, inhalation duration, *etc.*)*.* Further, in some examples, the inhaler may include a receiver configured to receive data (*e.g.,* the inhalation threshold or the canister actuation threshold) from the external device.

The pressure sensor may include a pressure port. The pressure port may be aligned with an opening between the medication canister and the housing when the pressure sensor is connected to the medication canister. In some examples, the pressure sensor (*e.g.,* and pressure port) is configured such that it does not extend into the inhaler housing when the pressure sensor is in the first position or the second position. For example, a gap (*e.g.,* physical space comprising ambient air) may exist between the pressure sensor (*e.g.,* and pressure port) and the flow channel of the inhaler when the pressure sensor is in the first position or is in the second position.

In some examples, the inhaler may include one or more additional sensors (*e.g.,* an orientation sensor, acoustic sensor, temperature sensor, mechanical switch, etc.). The processor may be configured to detect a shake event of the inhaler based on the pressure measurements received from the pressure sensor. The inhaler may include an orientation sensor configured to detect an orientation of the inhaler, and the processor may be configured to receive data from the orientation sensor and determine an orientation of the inhaler during an inhalation event. The processor may be configured to wake or turn on the pressure sensor in response to the reception of data from one or more different sensors, based on a mouthpiece cover of the inhaler being opened, and/or based on a switch on the inhaler being actuated.

In some examples, the inhaler may include a capillary tube connected to a sensor port of the pressure sensor, where the capillary tube is configured to extend from the pressure sensor into the housing.

Further, in some examples, the inhaler may be part of a system that includes the inhaler and an external device that is configured to receive data from the inhaler and provide feedback to the user *(e.g.,* via a display device of the external device) that indicates the detected events.

Finally, although described with reference to an inhaler, in some examples, the processor, pressure sensor, transmitter, memory, and power supply may be part of an accessory that is configured to be removably and replaceably attached to the inhaler. For examples, the accessory may be an electro-mechanical device that is configured to attached to an inhaler housing and/or a medication canister of an inhaler, and then later removed from the inhaler when, for example, the inhaler expires or is empty. An accessory would require additional user operation to physically attached the accessory to the inhaler, but would allow the accessory to be reused with multiple inhalers, thereby extending the useful life of the internal components.

With MDIs, for example, the timing between the user's inhalation and the actuation of the canister to release medicament can affect the amount of medicament that the user receives and how deep in the lungs the medicament is delivered. There is a need for a simple, accurate, and low costs inhaler or inhaler accessory that can provide an indication of the relative timing between the user's inhalation and the actuation of the medicament canister. For example, the user tends to receive a greater, and proper dose of medicament when the user inhales prior to actuating the canister. Existing inhalers may attempt to determine the relative timing of the user inhalation and the canister actuation, but these inhalers use multiple distinct sensors, where one sensor tracks the actuation of the canister and a second sensor detects the user's inhalation. But the use of two sensors can result in less accurate feedback, have increased production costs, and have additional points of failure. The devices described herein are configured to determine how well a user is inhaling and the relative timing between the user's inhalation and the canister actuation using one sensor (*e.g.,* a single pressure or acoustic sensor).

As noted, the timing between the user inhalation and canister actuation not only affects the amount of medicament that the user receives, but is also affects how deep in the lungs the medicament is delivered. Some active pharmaceutical ingredients are used to target specific portions of the user's respiratory track, and as such, it is desirable to deliver a maximum amount of each dose to that targeted portion of the user's respiratory track. For example, some medications may target the upper airways/respiratory track of the user, while others might target the lower airways/respiratory track of the user. In such instances, the inhaler described herein may be configured to generate the canister actuation notification during the user's inhalation (*e.g.,* based on inhaled volume and/or duration) to alert the user to actuate the canister to deliver a dose of medicament at a time that will increase the amount of the dose that is delivered to the targeted portion of the user's respiratory track. For instance, the inhaler may receive pressure measurements from a pressure sensor, determine one or more inhalation parameters based on the pressure measurements (*e.g.,* inhaled volume and/or inhalation duration), and then generate the canister actuation notification when the inhalation parameter reaches a particular threshold. The threshold may be dependent on the portion of the user's respiratory track that is targeted by the medicament of the inhaler. For example, when the medicament is used to target the upper airways, the notification may be generated later during the inhalation (*e.g.,* based on an inhalation parameter having a larger value) to ensure that a greater portion of the medication is provided to the upper airways, and less is delivered to the lower airways of the user. When the medicament is used to target the lower airways, the notification may be generated earlier during the inhalation (*e.g.,* based on an inhalation parameter having a smaller value) to ensure that a greater portion of the medication is provided to the lower airways of the user.

### BRIEF DESCRIPTION OF THE DRAWINGS

Fig. 1 is a block diagram of an example inhaler.
Fig. 2 is a diagram of a system that includes an inhaler and an external device.
Fig. 3A is an example block diagram that illustrates the location of an electronic module of an inhaler before a canister of the inhaler is actuated to dispense a dose of medicament.
Fig. 3B is an example block diagram that illustrates the location of an electronic module of an inhaler when a canister of the inhaler is actuated to dispense a dose of medicament.
FIG. 3C is an example block diagram that illustrates a top down perspective of an inhaler with the canister removed.
Fig. 4 is a graph of two example airflow rate versus pressure profiles.
Fig. 5A is a diagram of an example flow rate calculated by an electronics module of an inhaler, for example, when the user stops inhaling at the same time that they release a medicament canister of the inhaler.
Fig. 5B is a diagram of an example flow rate calculated by an electronics module of an inhaler, for example, when the user releases the medicament canister of the inhaler before they stop inhaling through the mouthpiece.
Fig. 6 is a diagram of an example inhaler that includes an electronic module that includes a capillary tube.
FIG. 7 is a diagram of an example system including two inhalation devices, an external device, a public and/or private network, a health care provider, and a third party computer or server.
Fig. 8 is a flowchart of an example procedure that is performed by an inhaler.

### DETAILED DESCRIPTION

It should be understood that the detailed description and specific examples, while indicating exemplary embodiments of the apparatus, systems and methods, are intended for purposes of illustration only and are not intended to limit the scope of the disclosure. These and other features, aspects, and advantages of the apparatus, systems and methods of the present disclosure will become better understood from the following description, appended claims, and accompanying drawings. It should be understood that the Figures are merely schematic and are not drawn to scale. It should also be understood that the same reference numerals are used throughout the figures to indicate the same or similar parts.

The present disclosure describes devices, systems and methods for sensing, tracking processing, and/or presenting usage conditions and parameters associated with a drug delivery device, such as an inhaler. The devices, systems and methods are described in the context of a metered-dose inhaler (MDI) (*e.g.,* also referred to herein as an inhaler) for delivering medication into a user's lungs. However, the described solutions are equally applicable to other drug delivery devices, such as an injector, a dry-powder inhaler, a nebulizer, a transdermal patch, or an implantable.

Asthma and COPD are chronic inflammatory disease of the airways. They are both characterized by variable and recurring symptoms of airflow obstruction and bronchospasm. The symptoms include episodes of wheezing, coughing, chest tightness and shortness of breath.

The symptoms are managed by avoiding triggers and by the use of medicaments, particularly inhaled medicaments. The medicaments include inhaled corticosteroids (ICSs) and bronchodilators.

Inhaled corticosteroids (ICSs) are steroid hormones used in the long-term control of respiratory disorders. They function by reducing the airway inflammation. Examples include budesonide, beclomethasone (dipropionate), fluticasone (propionate or furoate), mometasone (furoate), ciclesonide and dexamethasone (sodium). Parentheses indicate preferred salt or ester forms. Particular mention should be made of budesonide, beclomethasone and fluticasone, especially budesonide, beclomethasone dipropionate, fluticasone propionate and fluticasone furoate.

Different classes of bronchodilators target different receptors in the airways. Two commonly used classes are β2-agonists and anticholinergics.

β2-Adrenergic agonists (or "β2-agonists") act upon the β2-adrenoceptors which induces smooth muscle relaxation, resulting in dilation of the bronchial passages. They tend to be categorized by duration of action. Examples of long-acting β2-agonists (LABAs) include formoterol (fumarate), salmeterol (xinafoate), indacaterol (maleate), bambuterol (hydrochloride), clenbuterol (hydrochloride), olodaterol (hydrochloride), carmoterol (hydrochloride), tulobuterol (hydrochloride) and vilanterol (triphenylacetate). Examples of short-acting β2-agonists (SABA) are albuterol (sulfate) and terbutaline (sulfate). Particular mention should be made of formoterol, salmeterol, indacaterol and vilanterol, especially formoterol fumarate, salmeterol xinafoate, indacaterol maleate and vilanterol triphenylacetate.

Typically short-acting bronchodilators provide a rapid relief from acute bronchoconstriction (and are often called "rescue" or "reliever" medicines), whereas long-acting bronchodilators help control and prevent longer-term symptoms. However, some rapid-onset long-acting bronchodilators may be used as rescue medicines, such as formoterol (fumarate). Thus, a rescue medicine provides relief from acute bronchoconstriction. The rescue medicine is taken as-needed/prn (pro re nata). The rescue medicine may also be in the form of a combination product, e.g. ICS-formoterol (fumarate), typically budesonide-formoterol (fumarate) or beclomethasone (dipropionate)-formoterol (fumarate). Thus, the rescue medicine is preferably a SABA or a rapid-acting LABA, more preferably albuterol (sulfate) or formoterol (fumarate), and most preferably albuterol (sulfate).

Anticholinergics (or "antimuscarinics") block the neurotransmitter acetylcholine by selectively blocking its receptor in nerve cells. On topical application, anticholinergics act predominantly on the M3 muscarinic receptors located in the airways to produce smooth muscle relaxation, thus producing a bronchodilatory effect. Examples of long-acting muscarinic antagonists (LAMAs) include tiotropium (bromide), oxitropium (bromide), aclidinium (bromide), umeclidinium (bromide), ipratropium (bromide) glycopyrronium (bromide), oxybutynin (hydrochloride or hydrobromide), tolterodine (tartrate), trospium (chloride), solifenacin (succinate), fesoterodine (fumarate) and darifenacin (hydrobromide). Particular mention should be made of tiotropium, aclidinium, umeclidinium and glycopyrronium, especially tiotropium bromide, aclidinium bromide, umeclidinium bromide and glycopyrronium bromide.

A number of approaches have been taken in preparing and formulating these medicaments for delivery by inhalation, such as via a dry powder inhaler (DPI), a pressurized metered dose inhaler (pMDI) or a nebulizer.

According to the GINA (Global Initiative for Asthma) Guidelines, a step-wise approach is taken to the treatment of asthma. At step 1, which represents a mild form of asthma, the patient is given an as needed SABA, such as albuterol sulfate. The patient may also be given an as-needed low-dose ICS-formoterol, or a low-dose ICS whenever the SABA is taken. At step 2, a regular low-dose ICS is given alongside the SABA, or an as-needed low-dose ICS-formoterol. At step 3, a LABA is added. At step 4, the doses are increased and at step 5, further add-on treatments are included such as an anticholinergic or a low-dose oral corticosteroid. Thus, the respective steps may be regarded as treatment regimens, which regimens are each configured according to the degree of acute severity of the respiratory disease.

COPD is a leading cause of death worldwide. It is a heterogeneous long-term disease comprising chronic bronchitis, emphysema and also involving the small airways. The pathological changes occurring in patients with COPD are predominantly localized to the airways, lung parenchyma and pulmonary vasculature. Phenotypically, these changes reduce the healthy ability of the lungs to absorb and expel gases.

Bronchitis is characterized by long-term inflammation of the bronchi. Common symptoms may include wheezing, shortness of breath, cough and expectoration of sputum, all of which are highly uncomfortable and detrimental to the patient's quality of life. Emphysema is also related to long-term bronchial inflammation, wherein the inflammatory response results in a breakdown of lung tissue and progressive narrowing of the airways. In time, the lung tissue loses its natural elasticity and becomes enlarged. As such, the efficacy with which gases are exchanged is reduced and respired air is often trapped within the lung. This results in localized hypoxia, and reduces the volume of oxygen being delivered into the patient's bloodstream, per inhalation. Patients therefore experience shortness of breath and instances of breathing difficulty.

Patients living with COPD experience a variety, if not all, of these symptoms on a daily basis. Their severity will be determined by a range of factors but most commonly will be correlated to the progression of the disease. These symptoms, independent of their severity, are indicative of stable COPD and this disease state is maintained and managed through the administration of a variety drugs. The treatments are variable, but often include inhaled bronchodilators, anticholinergic agents, long-acting and short-acting β2-agonists and corticosteroids. The medicaments are often administered as a single therapy or as combination treatments.

Patients are categorized by the severity of their COPD using categories defined in the GOLD Guidelines (Global Initiative for Chronic Obstructive Lung Disease, Inc.). The categories are labelled A-D and the recommended first choice of treatment varies by category. Patient group A are recommended a short-acting muscarinic antagonist (SAMA) prn or a short-acting β2-aginist (SABA) prn. Patient group B are recommended a long-acting muscarinic antagonist (LAMA) or a long-acting β2-aginist (LABA). Patient group C are recommended an inhaled corticosteroid (ICS) + a LABA, or a LAMA. Patient group D are recommended an ICS + a LABA and/or a LAMA.

Patients suffering from respiratory diseases like asthma or COPD suffer from periodic exacerbations beyond the baseline day-to-day variations in their condition. An exacerbation is an acute worsening of respiratory symptoms that require additional therapy, i.e. a therapy going beyond their maintenance therapy.

For asthma, the additional therapy for a moderate exacerbation are repeated doses of SABA, oral corticosteroids and/or controlled flow oxygen (the latter of which requires hospitalization). A severe exacerbation adds an anticholinergic (typically ipratropium bromide), nebulized SABA or IV magnesium sulfate.

For COPD, the additional therapy for a moderate exacerbation are repeated doses of SABA, oral corticosteroids and/or antibiotics. A severe exacerbation adds controlled flow oxygen and/or respiratory support (both of which require hospitalization). An exacerbation within the meaning of the present disclosure includes both moderate and severe exacerbations.

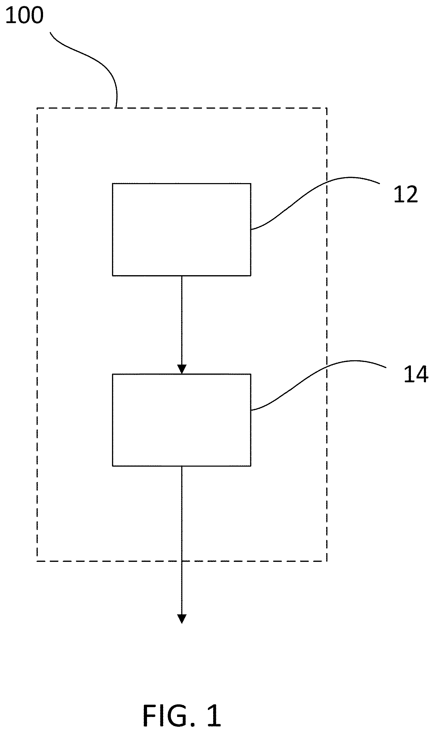

Fig. 1 shows a block diagram of an inhaler 100 according to an example. The inhaler 100 comprises a use determination system 12 that detects usage events. For example, a usage event may include usage of the inhaler 100, such as a detection that the inhaler 100 is being handled by the user, a priming of a dose of medicament in the inhaler, such as the shaking of the inhaler 100 (*e.g*., when the inhaler 100 is a MDI), the recording of an inhalation through the inhaler 100 by a user (*e.g.,* an inhalation event), a movement of a mouthpiece cover of the inhaler 100 from a closed to an open position, the actuation of a switch of the inhaler or a twist of a cap of the inhaler that advances a blister strip or prepares a dose of medicament, and/or any combination thereof.

Each usage event may include one or more usage parameters that are determined by the use determination system 12. Usage parameters may include any number of parameters indicative of a usage event at the inhaler 100 by a user. For example, usage parameters may include the duration of the shaking of the inhaler 100, the orientation of the inhaler 100 during an inhalation, the coordination of the actuation of a medication canister of the inhaler with an inhalation by a user, the temperature or humidity of the inhaler during an inhalation, and/or any combination of inhalation parameters (*e.g.,* flow rate, duration, *etc*.) that are measured during an inhalation event. The usage parameters may be measured by the use determination system 12, for example, in response to detecting a given usage event. Specifically, and as further described herein, the use determination system 10 may measure one or more inhalation specific usage parameters in response to detecting an inhalation event. The usage parameters measured by the use determination system 12 for an inhalation event, also referred to herein as inhalation parameter(s) (*e.g.,* as the relevant device is an inhaler), may include one or more of: a peak inspiratory/inhalation flow (PIF), an inhalation volume, a time to peak inhalation flow, and/or an inhalation duration.

The inhaler 100 may include a transmission module 14. The transmission module 14 may receive the usage parameter(s), as represented in Fig. 1 by the arrow between the block representing the use determination system 12 and the block representing the transmission module 14. The transmission module 14 encrypts data based on the value(s) of the usage parameter, and transmits the encrypted data, as represented in Fig. 1 by the arrow pointing away from the transmission module 14 block. The transmission of the encrypted data by the transmission module 14 may, for example, be wireless transmission to an external device, such as a smart phone or tablet. In examples where the transmission module 14 is configured to transmit the respective encrypted data wirelessly, the transmission module 14 may include a transceiver configured to implement any suitable wireless transmission protocol, such as via Wi-Fi, Wi-MAX, Bluetooth^{®}, Bluetooth^{®} Smart, ZigBee, near field communication (NFC), cellular communication, television white space (TVWS) communication, or any combination thereof.

Although examples described herein may refer to a transceiver, the transceiver may be configured to transmit, but not receive, data (e.g., a transmitter but not a receiver). The transceiver may include one or more semiconductor chips, integrated circuits, and/or the like configured to implement the logic and procedures of the communication protocol. The transceiver may include radio frequency (RF) hardware such as amplifier(s), oscillator(s), modulator circuit(s), antenna(s), antenna tuner(s), and/or the like in order to transmit signals wirelessly using the communication protocol. The RF hardware may be implemented in whole or in part on the semiconductor chip(s), integrated circuit(s), and/or the like configured to implement the logic and procedures of the communication protocol.

In some examples, the transmission module 14 is configured to transmit and/or receive data via Bluetooth^{®}. Bluetooth^{®} may be used because the relatively low energy associated with transmitting and receiving may preserve the battery life of the respective inhaler. Moreover, no internet connection need be established in order for the respective encrypted data to be transmitted.

The use determination system 12 may comprise a suitable processor, memory, and power source (*e.g.,* battery) configured to perform the functions described herein for the processing module. For example, the processor may be a general purpose processor programmed with computer executable instructions for implementing the functions of the use determination system 12. The processor may be implemented using a microprocessor or microcontroller configured to perform the functions of the use determination system 12. The processor may be implemented using an embedded processor or digital signal processor configured to perform the functions of the use determination system 12.

As described herein, a usage parameter may, for example, comprise an indication of a use of the respective inhaler. In a relatively simple implementation, the at least one value may indicate whether a usage event has been detected by the use determination system 12 and comprise "TRUE" when use of, for example an inhalation using, the inhaler 100 has been determined, or "FALSE" when no such use of the inhaler 100 is determined.

The use determination system 12 may include one or more of a switch configured to detect usage of inhaler 100, one or more sensors configured to detect use of inhaler 100, one or more buttons configured to be depressed upon use of inhaler 100, and/or the like. As such, a usage parameter may be one or more measurements performed by the use determination system, such as a count of the number of uses inhaler 100, a measure of airflow of inhaler 100, a detection of the canister of the inhaler 100 being actuated, and/or another measurement indicating the usage of the medicament of inhaler 100. The use determination system 12 may register each inhalation in different manners and/or based on additional or alternative feedback. For example, the use determination system 12 may be configured to register an inhalation by the user when the feedback from a suitable sensor indicates that an inhalation by the user has occurred, for example when a pressure measurement or flow rate exceeds a predefined threshold associated with a successful inhalation, when a medication canister of the inhaler is actuated, *etc.*

More generally, the use determination system 12 may comprise a sensor for detecting a parameter relating to airflow during inhalation of the respective medicament performed by the user. In other words, the usage parameter may comprise a parameter relating to airflow during inhalation of the medicament. The at least one value may thus, for example, comprise a numerical value relating to the detected inhalation parameter.

The sensor(s) of the use determination system 12 may include any combination of a pressure sensor, an acoustic sensor, a flow sensor, an accelerometer, and/or a touch sensitive device (*e.g.,* a capacitive touch sensitive circuit with capacitive touch sensitive pads). Such a sensor may be an alternative to or in addition to the abovementioned mechanical switch. The pressure sensor may be a differential pressure sensor or a barometric/absolute pressure sensor.

The use determination system 12 may use feedback from a sensor, such as an accelerometer and/or touch sensitive device to change power states. For example, the use determination system 12 may reside in a low power state, such as an off state or a sleep state, when not in use. During the low power state, any combination of sensors, the transmission module 14, and/or various pins on the processor may be off (*e.g.,* other than the sensor and associated pin on the processor that is used to trigger the use determination system 12 to switch power states). The use determination system 12 may switch from the low power state to an on state in response to receiving feedback from a sensor, such as feedback from an accelerometer that indicates that the user is shaking the inhaler (*e.g.,* an MDI inhaler) or feedback from a touch sensitive device that indicates that the user has the inhaler in their hand, for example. The use determination system 12 may turn on the transmission module 14 and/or the pressure sensor, and/or begin receiving measurements from the pressure sensor in response to switching from a low power state, such as an off state or a sleep state, to an on state.

The use determination system 12 may receive pressure measurements from a pressure sensor, and may calculate one or more inhalation parameters based on the pressure measurements. The inhalation parameter may be, for example, at least one of flow rate, a PIF, an inhalation volume, a time to peak inhalation flow, and/or an inhalation duration. As detailed further herein, the use determination system 12 may use the inhalation parameter to classify a respective usage event and/or inhalation event (*e.g.,* a good inhalation event, a fair inhalation event, a low/no inhalation event, an exhalation event, a possible air vent block event, *etc*.). Further, in some examples, the use determination system 12 may determine and store a flow profile (e.g., a series of consecutive pressure measurements) associated with a usage event. As described herein, these inhalation parameters may be used to assess the user's compliance, which may provide valuable insight into the state of the user's respiratory disease (*e.g.,* the likelihood that the patient is to experience an exacerbation event) and/or the patient's sensitivity to current or changing environmental conditions (*e.g.,* heat, air quality, humidity, *etc*.)*.* Further, the use determination system 12 may timestamp and/or geotag the usage event, error event, and/or usage parameters (*e.g.,* using a real-time stamp or a relative time stamp that begins when the user first operates the inhaler).

A pressure sensor may be particularly suitable for measuring the parameter, since the airflow during inhalation by the user may be monitored by measuring the associated pressure changes. The pressure sensor may be located external to a flow pathway through which air and the medicament is drawn by the user during inhalation. An inhalation may be associated with a decrease in the pressure in the airflow channel of the inhaler relative to when no inhalation is taking place. The point at which the pressure change is at its greatest may correspond to the peak inhalation flow. The pressure sensor may detect this point in the inhalation. Alternative ways of measuring the parameter, such as via a suitable flow sensor, can also be used.

The pressure change associated with an inhalation may alternatively or additionally be used to determine an inhalation volume. This may be achieved by, for example, using the pressure change during the inhalation measured by the pressure sensor to first determine the flow rate over the time of the inhalation, from which the total inhaled volume may be derived.

The pressure change associated with an inhalation may alternatively or additionally be used to determine an inhalation duration. The time may be recorded, for example, from the first decrease in pressure measured by the pressure sensor, coinciding with the start of the inhalation, to the pressure returning to a pressure corresponding to no inhalation taking place.

The inhalation parameter may alternatively or additionally include the time to peak inhalation flow. This time to peak inhalation flow parameter may be recorded, for example, from the first decrease in pressure measured by the pressure sensor, coinciding with the start of the inhalation, to the pressure reaching a minimum value corresponding to peak flow.

Further, in some examples, the inhalation parameter may be a flow profile that includes a plurality of measurements from the pressure sensor taken over time (*e.g.,* which may include one or more of a peak inhalation flow, an inhalation volume, a time to peak inhalation flow, and/or an inhalation duration). For example, the use determination system 12 may turn on the pressure sensor and/or begin receiving measurements from the pressure sensor in response to switching from a low power state, such as an off state or a sleep state, to an on state. The use determination system 12 may determine (*e.g.,* calculate) a flow profile using the received pressure measurements.

The use determination system 12 may compare the measurements received from the pressure sensor (*e.g.,* the flow profile) to one or more thresholds and determine whether the measurements are indicative of an inhalation by the user. For instance, the use determination system 12 may determine that the measurements are indicative of an inhalation by the user when a pressure measurement exceeds a predetermined value or is within a predetermine range that is indicative of peak inhalation by a user, the pressure measurements indicate a slope that is indicative of inhalation (*e.g.,* there is a change in pressure over time that is indicative of inhalation), the pressure measurements indicate an inhalation duration that is within a predetermined time range, and/or the like. After determining that the measurements are indicative of an inhalation by the user, the use determination system 12 may store a plurality of the measurements received from the pressure sensor as a flow profile associated with the inhalation event.

Further, in some examples, the use determination system 12 may include a sensor, and the use determination system 12 may be configured to determine the position of the sensor and/or the medication canister of the inhaler 100 while the user inhales through the inhaler 100. The use determination system 12 may be configured to detect whether an inhalation by a user through the inhaler 100 occurs prior a medication canister of the inhaler 100 being depressed to release medication based on measurements received from the sensor (*e.g.,* pressure measurements from a pressure sensor, acoustic measurements from an acoustic sensor, or flow measurements from a flow sensor). For example, the pressure sensor (*e.g.,* or a sensor port of the pressure sensor) may be attached to or moveable in relation to any movement of the medication canister. Further, the inhaler 100 may include an airflow channel that, for example, resides between a mouthpiece of the inhaler and an air inlet of the inhaler. As such, when the canister is depressed either by the user's actuation or inhalation (*e.g.,* in the case of a breath actuated MDI), the pressure sensor and/or sensor port of the pressure sensor is configured to move relative to the flow channel of the inhaler 100. Accordingly, (*e.g.,* while a user is inhaling through the inhaler 100) the pressure sensor may be configured to measure a first pressure when the pressure sensor is in the first position (*e.g.,* prior to canister actuation) relative to the flow channel of the inhaler 100, and a distinguishable second pressure when the canister is in the second position (*e.g.,* a depressed position to release a dose of medication) relative to the flow channel of the inhaler 100. The use determination system 12 may determine that the canister moved positions when the pressure measurements exceed a slope threshold.

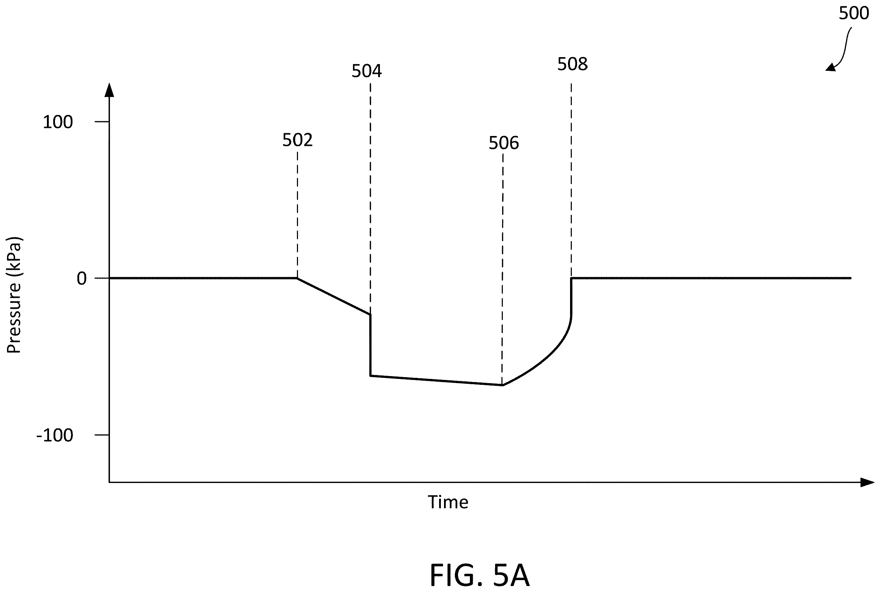

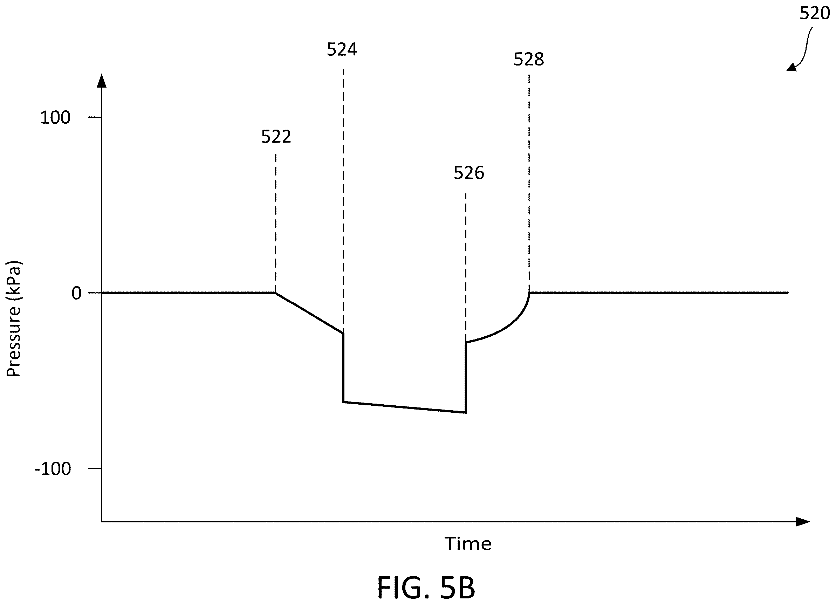

For instance, as described in more detail herein, while the user is inhaling through the inhaler 100, the pressure measurements may change by a distinguishable amount (*e.g.,* a step, such as shown in the examples of FIG. 5A and 5B) when the relative position of the pressure sensor to the flow channel changes upon actuation of the canister. The use determination system 12 may be configured to detect whether an inhalation by a user through the inhaler 100 occurs prior a medication canister of the inhaler 100 being depressed to release medication based on a distinguishable change is pressure measurements received from the pressure sensor. Further, as noted above, the use determination system 12 may be configured to determine the position of the pressure sensor and/or the medication canister based on the received pressure measurements. Although primarily described with reference to the use determination system 12 determining the position of the canister and/or the relative timing of the canister actuation based on a comparison of the pressure measurements to a slope threshold, it should be appreciated that in other examples the use determination system 12 may be other thresholds, such as a change in flow rate over time (*e.g.,* a flow rate slope threshold), an absolute pressure measurement value, and/or the like. Further, although described with reference to a pressure sensor, an acoustic sensor or flow sensor may be used.

In some examples, the pressure sensor is configured such that it does not extend into the inhaler housing when the pressure sensor is in the first position and/or the second position. For example, the pressure sensor may be configured such that a gap exists between the pressure sensor (*e.g.,* and pressure sensor port) and the flow channel of the inhaler when the pressure sensor is in the first position and/or the second position. The pressure sensor may include a sensor port that is aligned with an opening between the medication canister and the inhaler housing when the pressure sensor is connected to the medication canister. However, in some examples, the sensor port does not extend into or between the inhaler housing and the canister. Further, in some examples, the pressure sensor may be pneumatically coupled to the flow channel, but the pneumatic coupling may be rather weak (*e.g.,* due to the gap between the sensor port and the inhaler housing). For example, the pressure sensor may not be in an airtight or substantially airtight configuration with respect to the flow channel. Since, in some examples, the pressure sensor may be weakly pneumatically coupled to the flow channel of the inhaler 100, the pressure measurements may change by a distinguishable amount when the relative position of the pressure sensor to the flow channel changes upon actuation of the canister.

The use determination system 12 and/or the pressure sensor may be configured with a first flow rate calibration curve for when the pressure sensor is in the first position, and a second flow rate calibration curve for when the pressure sensor is in the second position. Further, in some examples, the use determination system 12 and/or the pressure sensor is calibrated based on only one of the first position or the second position (*e.g.,* which may ensure the distinguishable change in pressure measurements when the pressure sensor changes position relative to the flow channel).

The use determination system 12 may be configured to compare the received pressure measurements (*e.g.,* or one or more inhalation parameters calculated using the received pressure measurements) to one or more thresholds to determine one or more events. For example, the use determination system 12 may be configured to compare the pressure measurements to an inhalation threshold, and determine (*e.g.,* generate) an inhalation event based on the pressure measurements exceeding the inhalation threshold. The inhalation threshold may be an absolute pressure value, a rate of change of the pressure measurements (*e.g.,* a slope threshold), and/or the like. The use determination system 12 may be configured to compare the pressure measurements to a canister actuation threshold, and determine (*e.g.,* generate) a canister actuation event based on the pressure measurements exceeding the canister actuation threshold. The canister actuation threshold may be a rate of change of the pressure measurements (*e.g.,* a slope threshold), or an absolute value. The canister actuation threshold may be larger than the inhalation event (*e.g.,* a greater rate of change, or slope threshold, or a larger absolute negative pressure value), for example since the actuation of the canister may bring the pressure sensor closer to the flow channel.

The use determination system 12 may be configured to compare the pressure measurements to a canister released threshold, and detect the movement of the canister back to the first position based on the pressure measurements falling exceeding a canister release threshold. For example, the canister release threshold may be slope threshold that indicates a positive rate of change of the pressure measurements that indicates that the canister has moved back to the unactuated position, or may be an absolute pressure value. The use determination system 12 may generate a canister released event when the rate of change of the pressure measurements exceeds the canister release threshold. The use determination system 12 may be configured to determine a duration of an inhalation event based on the amount of time that the pressure measurements (*e.g.,* flow rates) exceed the inhalation threshold. Alternatively, the duration of the inhalation event may start at the time of the canister actuation event and ends when the flow rates fall below the inhalation threshold.

The use determination system 12 may be configured to generate one or more error events based on feedback from the pressure sensor. For example, the use determination system 12 may be configured to generate a late inhalation error event when the detection of an inhalation occurs after the medication canister is depressed. Alternatively or additionally, the use determination system 12 may be configured to generate a late actuation error event when the pressure measurements (*e.g.,* flow rates) exceed a threshold for a period of time that exceeds a duration threshold prior to the detection of the canister being actuated. For example, the use determination system 12 may determine that the user inhales for too long of a period of time prior to the canister being actuated, and in response, generate the late actuation error event. The inhaler may generate an on-time inhalation event if the user's inhalation occurred within a predetermined time period before the canister was actuated. The use determination system 12 may be configured to send the error events, via transmission module 14, to an external device for display via a user interface of the external device (*e.g.,* a smartphone, tablet, PC, etc.). Alternatively or additionally, the use determination system 12 may send the pressure measurements to the external device, and the external device may generate any combination of the events described herein (*e.g.,* the inhalation event, the canister actuation event, the canister released event, any of the error events), for example, and display an indication of the error event on the user interface of the external device. Further, the use determination system 12 and/or the external device may timestamp and/or geotag the events and/or usage parameters (*e.g.,* using a real-time stamp or a relative time stamp that begins when the user first operates the inhaler).

Further, in some examples, the use determination system 12 may be configured to generate a canister actuation notification to indicate to the user when to actuate the canister. The inhaler 100 may include an indicator, such as a light, speaker, a display, a vibrating element, etc., that is configured to generate a notification or provide feedback to the user. The use determination system 12 may be configured to calculate inhalation volume and/or inhalation duration based on the received pressure measurement. The use determination system 12 may determine when the pressure measurements (*e.g.,* flow rates) indicate that the inhaled volume and/or duration of an inhalation exceed a canister notification threshold, and in response, generate the canister actuation notification via the indicator to indicate to the user to actuate the canister. As such, the use determination system 12 may be configured to alert the user when to actuate the canister to assist the user in receiving the proper amount of medication and/or to assist in ensuring delivery of the medication to the proper portion of the respiratory system (*e.g.,* the upper airways/respiratory track or the lower airways/respiratory track). The canister notification threshold may be based on the portion of the user's respiratory track that is targeted by the medicament of the inhaler 100. In some instances, the active pharmaceutical ingredient of the medication may be used to target the upper airways of the user. In such instances, the use determination system 12 may be configured to generate the canister actuation notification at a time during the user's inhalation (*e.g.,* based on inhaled volume and/or duration) to ensure that the active pharmaceutical ingredient of the medication is delivered to the upper airways of the user. For example, the notification may be generated later than normal during the inhalation to ensure that a greater portion of the medication is provided to the upper airways, and less is delivered to the lower airways of the user.

As noted above, the use determination system 12 may be configured to detect a user's exhalation through the inhaler 100. Further, the use determination system 12 may be configured to detect the duration of time that a user holds their breath based on measurements received from the sensor (*e.g.,* the pressure sensor or acoustic sensor). For example, the use determination system 12 may detect the beginning of the user's inhalation when the received pressure measurements exceed an inhalation threshold (*e.g.,* a slope in pressure measurements that is indicative of a user's inhalation), and detect when the user stops inhaling (*e.g.,* has inhaled completely) based on the received pressure measurements (*e.g.,* when the pressure measurements return to or fall below a particular value). The use determination system 12 may detect when the user exhales their breath based on received pressure measurement assuming, for example, that the user exhales through the inhaler 100. The use determination system 12 may determine the time period that the user held their breath based on the time difference between the end of the user's inhalation (*e.g.,* or, in some examples, the start) and the detection of the user's exhalation. The time that the user's held their breath can be indicative of the amount of medicament that the user receives during their inhalation, as typically, a longer breath hold results in more medicament being delivered into the user's respiratory system.

Alternatively or additionally, the use determination system 12 may, for instance, comprise a mechanical switch configured to be actuated prior to, during, or after use of the inhaler. The mechanical switch may indicate that a dose of medicament has been primed and is ready for inhalation (e.g., such as by metering a dose from a hopper, advancing and/or opening a blister pack, breaking open and/or spinning a pill comprising medicament, etc.). In a non-limiting example, the inhaler 100 comprises a medicament reservoir (not visible in Fig. 1) that houses medicament (*e.g.,* a medication canister, a blister strip, a hopper, *etc*.), and/or a dose metering assembly (not visible in Fig. 1) configured to meter a dose of the medicament from the reservoir. The dose metering assembly may include a bellows that is configured to meter a dose of dry powder medicament from a hopper, a blister strip advancement and opening mechanism, a chamber configured to open a pill of dry powder medicament, a metering valve and an actuator seat/nozzle, and/or the like. The use determination system 12 may be configured to register the metering of the dose by the dose metering assembly, each metering being thereby indicative of the inhalation performed by the user using the inhaler 100. Accordingly, the inhaler 100 may be configured to monitor the number of inhalations of the medicament, since the dose should be metered via the dose metering assembly before being inhaled by the user. Alternatively or additionally, the use determination system 12 may be configured to register a usage event based on the metering of the dose by the dose metering assembly and register another usage event based on feedback from one or more sensors. In such examples, the use determination system 12 may be configured to correlate the timing and/or duration of each of the usage events, which for example, may be used to determine whether the user proper primed the inhaler 100 and properly inhaled the dose of medicament, since both steps are valuable in determining whether a successful dose is received.

Although described with reference to the inhaler 100, in some examples, the use determination system 12 and the transmission module 14 may be part of an accessory that is configured to be removably and replaceably attached to an inhaler. For examples, the accessory may be an electro-mechanical device that is configured to attached to an inhaler housing and/or a medication canister of an inhaler, and then later removed from the inhaler when, for example, the inhaler expires or is empty. An accessory would require additional user operation to physically attached the accessory to the inhaler, but would allow the accessory to be reused with multiple inhalers, thereby extending the useful life of the internal components.

Further, although described with reference to a pressure sensor, in some examples, the inhaler may include an acoustic sensor instead of a pressure sensor. The acoustic sensor may experience similar changes in measured acoustic signals when the relative position of the sensor to the flow channel of the inhaler changes due to a canister actuation. Accordingly, the use determination system may be configured to detect whether an inhalation by a user through the inhaler 100 occurs prior a medication canister of the inhaler 100 being depressed to release medication based on a distinguishable change is acoustic measurements received from the acoustic sensor. Further, the use determination system 12 may be configured to determine the position of the acoustic sensor and/or the medication canister based on the received acoustic measurements.

Although described with reference to a pressure sensor, in some examples, the inhaler may include a flow sensor instead of a pressure sensor. The flow sensor may experience similar changes in measured flow signals when the relative position of the sensor to the flow channel of the inhaler changes due to a canister actuation. Accordingly, the use determination system may be configured to detect whether an inhalation by a user through the inhaler 100 occurs prior a medication canister of the inhaler 100 being depressed to release medication based on a distinguishable change is flow measurements received from the flow sensor. Further, the use determination system 12 may be configured to determine the position of the flow sensor and/or the medication canister based on the received flow measurements.

The memory of the use determination system 12 may comprise a computer-readable storage media or machine-readable storage media that maintains computer-executable instructions for performing one or more as described herein. For example, the memory may comprise computer-executable instructions or machine-readable instructions that include one or more portions of the procedures described herein. The use determination system 12 may access the instructions from memory for being executed to cause the use determination system 12 to operate as described herein, or to operate one or more other devices as described herein. The memory may comprise computer-executable instructions for executing configuration software. For example, the computer-executable instructions may be executed to perform the procedure 800 as described herein. Further, the memory may have stored thereon one or more settings and/or control parameters associated with the use determination system 12.

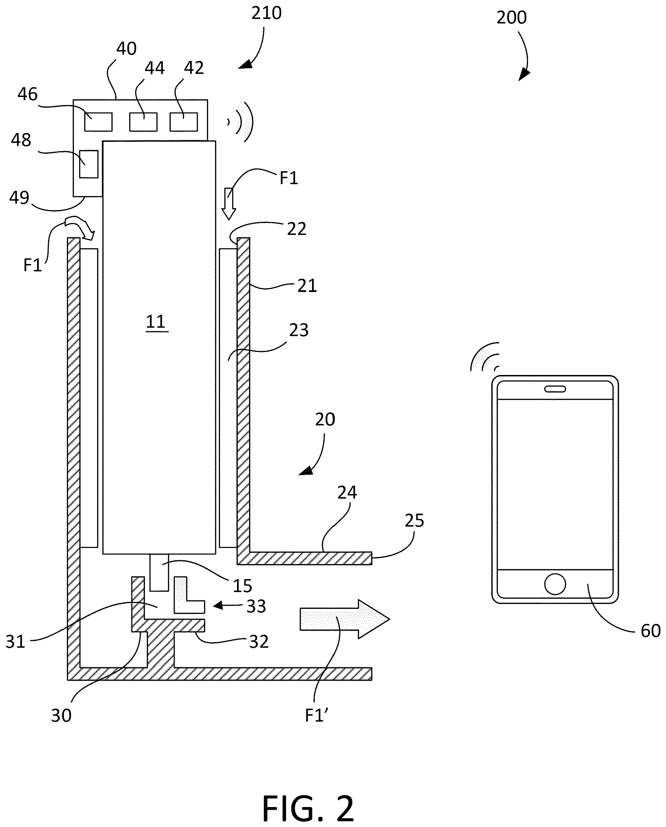

Fig. 2 is a diagram of a system 200 that includes an inhaler 210 and an external device 60. The inhaler 210 may be an example of the inhaler 100 of Fig. 1. The inhaler 210 may include an electronics module 40, and the electronics module 40 may include a use determination system (*e.g.,* the use determination system 12) and a transmission module (*e.g.,* the transmission module 14). Although described as part of the inhaler 210, the electronics module 40 may be incorporated as a removable and replaceable accessory that is attachable to the inhaler 210. Further, although illustrated as attached to the top of a medication canister 11 of the inhaler 210, the electronics module 40 may be attached to another portion of the medication canister 11 and/or otherwise attached to the inhaler 210 such that a pressure sensor of the electronics module moves relative to a flow channel of the inhaler 210 when medication is dispensed (*e.g.,* when the canister 11 is actuated).

The inhaler 210 may be a pMDI inhaler that includes a generally cylindrical medicament canister 11 that has at its lower end a metering valve for controlled release of single doses of a medicament through a hollow valve stem 15 of the inhaler 210. The metering valve may be actuated by pressing the valve stem 15 into canister 11. Further, in some examples, the inhaler 210 may be a breath actuated MDI, and in such instances, the metering value may be actuated by the valve stem 15 being pressed into the canister 11 in response to a user's inhalation.

The canister 11 may be received in an inhaler housing 20, which includes a mouthpiece 24. In the illustrated example of Fig. 2, the inhaler housing 20 is generally L-shaped, however in other examples, the inhaler housing 20 may take on other shapes, such as disk-shaped or cylindrical shaped. The upright leg of the L forms an upwardly open receiving portion for the canister 11. The receiving portion has the shape of a hollow cylinder extending upwardly to an upper end 22. The receiving portion is defined by a circumferential housing wall 21. At the open upper end 22 of the receiving portion, the housing wall 21 forms an annular end face. In some examples, the canister 11 is held in the receiving portion by a plurality of spacer ribs 23 extending radially inwardly from the housing wall 21. As such, the outer circumferential wall of the canister 11 is spaced from the housing wall 21 of the housing 20, and axially extending air channels are thus formed between the housing wall 21 and the drug container 11. These air channels form a portion of the airflow channel of the inhaler 210.

At the lower end of the receiving portion, the inhaler housing 20 includes a hollow socket 30. The valve stem 12 of the drug container 11 may be seated in the socket 30. The socket 30 defines a duct 31 leading from the exit of the valve stem 12 to a nozzle orifice 32. The nozzle orifice 32 opens out into the interior of the inhaler housing 20 in a lateral direction that is transverse to the cylinder axis of the receiving portion, but not necessarily perpendicular to the cylinder axis.

The transverse leg of the L-shaped housing 20 may extend in the same lateral direction as the nozzle orifice 32. At its far end, it forms the mouthpiece 24 for insertion into the mouth of a patient. The mouthpiece 24 is laterally open at its end 25. In some examples, the mouthpiece 24 has a flattened cross-sectional shape adapted to the anatomy of a human patient's mouth. This cross-sectional shape is generally different from the cross-sectional shape of the receiving portion.

The open upper end 22 of the receiving portion of the inhaler housing 20 together with the circumferential wall of the canister 11 forms an air inlet, allowing air to enter the inhaler 210. The open end 25 of the mouthpiece 24 forms an air outlet. An airflow path exists through the inhaler housing 20 between the air inlet and the air outlet (*e.g.,* the mouthpiece 24). The nozzle orifice 32 is disposed in this airflow path. Downstream from the nozzle orifice 32, the inhaler housing 20 may define a mixing zone 33 for mixing the airflow with the drug released from the canister 11.

In use, a user holds the inhaler 210 and applies his mouth to the mouthpiece 24. The user then inhales through the mouthpiece 24, thereby creating an air flow F1 through the inhaler housing 20 from the air inlet to the air outlet. After the patient has started inhaling through the mouthpiece 24, the canister 11 is pressed downwardly to release a dose of drug (*e.g.,* in response to a user's physical actuation of the canister 11 or through a breath actuated actuation mechanism of the inhaler 210). Due to the pressure in the canister 11, the medicament may be propelled through the duct 31 and the nozzle orifice 32 into the mixing zone 33, where it mixes with the airflow to form an aerosol flow F1', and is dispensed to the user.

As noted above, the electronics module 40 may include a use determination system (*e.g.,* the use determination system 12) and a transmission module (*e.g.,* the transmission module 14). For example, the electronics module 40 may include a processor 42, memory, a power source 44, a communication circuit 46 (*e.g.,* transmitter and/or receiver), a pressure sensor 48, and in some instances, one or more additional sensors, such as those described with reference to the inhaler 10 of Fig. 1. The processor 42 may be a general purpose processor programmed with computer executable instructions for implementing the functions of the electronics module 40. The processor 42 may be implemented using a microprocessor or microcontroller configured to perform the functions of the electronics module 40. The processor 42 may be implemented using an embedded processor or digital signal processor configured to perform the functions of the electronics module 40. In some examples, the power source 44 is a battery. Further, in some examples, the electronics module 40 may include any combination of a mechanical switch, an acoustic sensor, a flow sensor, an accelerometer, and/or a touch sensitive device (*e.g.,* a capacitive touch sensitive circuit with capacitive touch sensitive pads).

The memory of electronics module 40 may comprise a computer-readable storage media or machine-readable storage media that maintains computer-executable instructions for performing one or more as described herein. For example, the memory may comprise computer-executable instructions or machine-readable instructions that include one or more portions of the procedures described herein. The processor 42 may access the instructions from memory for being executed to cause the processor 42 to operate as described herein, or to operate one or more other devices as described herein. The memory may comprise computer-executable instructions for executing configuration software. For example, the computer-executable instructions may be executed to perform at least a portion of the procedure 800 as described herein. Further, the memory may have stored thereon one or more settings and/or control parameters associated with the electronics module 40.

The communication circuit 46 may include one or more semiconductor chips, integrated circuits, and/or the like configured to implement the logic and procedures of the communication protocol. The communication circuit 46 may include radio frequency (RF) hardware such as amplifier(s), oscillator(s), modulator circuit(s), antenna(s), antenna tuner(s), and/or the like in order to transmit signals wirelessly using the communication protocol. The RF hardware may be implemented in whole or in part on the semiconductor chip(s), integrated circuit(s), and/or the like configured to implement the logic and procedures of the communication protocol. In some examples, the communication circuit 46 is configured to transmit and/or receive data via Bluetooth^{®}. Bluetooth^{®} may be used because the relatively low energy associated with transmitting and receiving may preserve the battery life of the respective inhaler. Moreover, no internet connection need be established in order for the respective encrypted data to be transmitted.

The pressure sensor 48 may be a differential pressure sensor (*e.g.,* sometimes referred to as a relative pressure sensor) or a barometric pressure sensor (*e.g.,* sometimes referred to as an absolute pressure sensor). The pressure sensor 48 can, in particular, be a differential pressure sensor that is based on a flow measurement principle. A differential pressure sensor of this type has two sensor ports: a sensor inlet and a sensor outlet. A pressure difference between the sensor inlet and the sensor outlet causes a sensor gas flow through a sensor flow channel defined inside the differential pressure sensor. A flow-sensitive structure is arranged adjacent to the sensor flow channel for measuring a flow rate of the sensor gas flow through the sensor flow channel. Thus, a differential pressure sensor of this type essentially acts as a flow sensor that is configured to determine the pressure difference based on the determination of a flow rate through a flow channel between the sensor ports.

In some examples, when the pressure sensor 48 is a barometric pressure sensor, the processor 42 may receive consecutive measurements from the barometric pressure sensor to monitor a change or difference in the measurements that is indicative of a user's inhalation. For example, the processor 42 may be configured to receive a first measurement (or series of measurements, such as a rolling average) prior to the user's inhalation, and use this measurement(s) to calculate a tare value. The tare value may be an absolute baseline reference reading that is taken when the canister is at the first position (*e.g.,* an undepressed position) without a user's inhalation. The processor 42 may be configured to determine the pressure change due to the user's inhalation by calculating the tare value from subsequent pressure measurements received from the pressure sensor 48. Accordingly, the tare value may be used to determine a change in air pressure that may be indicative of the start of an inhalation event (e.g., a slope that exceeds a threshold indicative of inhalation, a flow rate that exceeds an inhalation threshold, *etc*.).

The electronics module 40 may include a pressure port 49. If a differential pressure sensor is used, one of the sensor ports of the sensor is pneumatically connected to the pressure port 49 in a fluid-tight manner, while a second pressure port is pneumatically connected to the environment of the electronics module 40 in a region that is not influenced by the air flow F1. To this end, in some examples, the electronics module 40 may include a reference port (not shown) in the form of an opening, the opening being arranged in a region of the electronics module 40 that faces away from the inhaler 210. The opening may cause the pressure inside the electronics module 40 to be equal to the pressure of the environment of the electronics module 40 in a region that is unaffected by the air flow F1. The sensor port that is not connected to the pressure port 49 can therefore be simply open towards the inside of the electronics module 40, without a fluid-tight connection being required between this sensor port and the reference port. If a barometric pressure sensor is used, then only a single port is needed since the barometric pressure sensor is referenced to atmospheric pressure.

Once the electronics module 40 turns on, the pressure sensor 48 may begin generating pressure measurements and the processor 42 may look for a change in the air pressure, as detected using the pressure sensor. In some examples, the processor 42 may analyze a rolling number of consecutive pressure measurements to determine a tare value, or dynamic zero value, to calibrate the pressure sensor 48 to the atmospheric pressure, and then use the tare value to determine a change in air pressure that may be indicative of the start of an inhalation event (e.g., a slope that exceeds a threshold indicative of inhalation, a flow rate that exceeds an inhalation threshold, *etc*.)*.*

The processor 42 may be configured to receive pressure measurements from the pressure sensor 48, and determine one or more usage events or usage parameters based on the pressure measurements. Alternatively or additionally, the processor 42 may be configured to detect usage events based on feedback from other sensors or switches of the inhaler 210. For example, a usage event may include usage of the inhaler 210, such as a detection that the inhaler 210 is being handled by the user, a priming of a dose of medicament in the inhaler, such as the shaking of the inhaler 210 (*e.g.,* when the inhaler 210 is a MDI), the recording of an inhalation through the inhaler 210 by a user (*e.g.,* an inhalation event), a movement of a mouthpiece cover of the inhaler 210 from a closed to an open position, the actuation of a switch of the inhaler or a twist of a cap of the inhaler that advances a blister strip or prepares a dose of medicament, and/or any combination thereof. Further, the processor 42 may timestamp and/or geotag the events and/or usage parameters (*e.g*., using a real-time stamp or a relative time stamp that begins when the user first operates the inhaler

Each usage event may include one or more usage parameters that are determined by the electronics module 40. Usage parameters may include any number of parameters indicative of a usage event at the inhaler 210 by a user. For example, usage parameters may include the duration of the shaking of the inhaler 210, the orientation of the inhaler 210 during an inhalation, the coordination of the actuation of a medication canister 11 of the inhaler with an inhalation by a user, the temperature or humidity of the inhaler 210 during an inhalation, and/or any combination of inhalation parameters (*e.g.,* flow rate, duration, *etc*.) that are measured during an inhalation event. The usage parameters may be measured by the processor 42, for example, in response to detecting a given usage event. Specifically, and as further described herein, the processor 42 may measure one or more inhalation specific usage parameters in response to detecting an inhalation event. The usage parameters measured by the processor 42 for an inhalation event, also referred to herein as inhalation parameter(s) (*e.g.,* as the relevant device is an inhaler), may include one or more of: a peak inspiratory/inhalation flow (PIF), an inhalation volume, a time to peak inhalation flow, and/or an inhalation duration. The processor 42 may send, via the communication circuit 46, usage events and/or usage parameters to the external device 60.

The processor 42 may receive pressure measurements from the pressure sensor 48, and may calculate one or more inhalation parameters based on the pressure measurements. The inhalation parameter may be, for example, at least one of flow rate, a PIF, an inhalation volume, a time to peak inhalation flow, and/or an inhalation duration. As detailed further herein, the processor 42 may use the inhalation parameter to classify a respective usage event and/or inhalation event (*e.g.,* a good inhalation event, a fair inhalation event, a low/no inhalation event, an exhalation event, a possible air vent block event, *etc*.)*.* Further, in some examples, the processor 42 may determine and store a flow profile (e.g., a series of consecutive pressure measurements) associated with a usage event. As described herein, these inhalation parameters (*e.g.,* also referred to herein as inhalation parameters) may be used to assess the user's compliance, which may provide valuable insight into the state of the user's respiratory disease (*e.g.,* the likelihood that the patient is to experience an exacerbation event) and/or the patient's sensitivity to current or changing environmental conditions (*e.g.,* heat, air quality, humidity, *etc*.)*.* Further, although described at times in reference to the received pressure measurements, in some examples, the processor 42 may convert the received pressure measurements to a corresponding flow rate, and use the corresponding flow rates to determine one or more of the events and/or parameters described herein.

The usage information determined by the processor 42 include a classification of one or more inhalation events. For example, if the peak inhalation flow is between 0 and a lower flow rate (*e.g.,* 30 liters per minute), the inhalation event is classified as "low inhalation" (less than or equal to 30 liters per minute) or as "no inhalation". If the peak inhalation flow is greater than the lower flow rate (*e.g.,* 30 LPM) and less than or equal to an intermediate flow rate (*e.g.,* 45 liters per minute), the inhalation event is classified as "fair". If the peak inhalation flow is greater than the intermediate flow rate (*e.g.,* 45 LPM) and less than or equal to an upper flow rate (*e.g.,* 200 liters per minute), the inhalation event is classified as a "good inhalation". If the peak inhalation flow is above the upper flow rate (*e.g.,* 200 liters per minute), the inhalation event is classified as an excessive inhalation event and/or a "possible air vent block".

The inhalation event may be classified as an "exhalation", for example, if the processor 42 receives pressure measurements that indicate a positive change in pressure. The classification of inhalation parameter(s) as a respective inhalation event (e.g., a good inhalation event, a fair inhalation event, a low/no inhalation event, an exhalation event, a possible air vent block event, etc.) may be based on or result from calculations performed at the processor 42 and/or at the external device 60. Finally, it should be appreciated that the flow rates provided are examples, and that the classification of inhalation events may be based on a different ranges of flow rates than those provided herein.

An inhalation may be associated with a decrease in the pressure in the airflow channel of the inhaler 210 relative to when no inhalation is taking place. The point at which the pressure change is at its greatest may correspond to the peak inhalation flow. The processor 42 may detect this point in the inhalation. Alternative ways of measuring the parameter, such as via a suitable flow sensor, can also be used.

The pressure change associated with an inhalation may alternatively or additionally be used to determine an inhalation volume. This may be achieved by, for example, using the pressure change during the inhalation measured by the pressure sensor to first determine the flow rate over the time of the inhalation, from which the total inhaled volume may be derived.

The pressure change associated with an inhalation may alternatively or additionally be used to determine an inhalation duration. The time may be recorded, for example, from the first decrease in pressure measured by the pressure sensor, coinciding with the start of the inhalation, to the pressure returning to a pressure corresponding to no inhalation taking place.

The inhalation parameter may alternatively or additionally include the time to peak inhalation flow. This time to peak inhalation flow parameter may be recorded, for example, from the first decrease in pressure measured by the pressure sensor, coinciding with the start of the inhalation, to the pressure reaching a minimum value corresponding to peak flow.

The pressure port 49 of the electronics module 40 may be aligned with the open upper end 22 of the housing 20. The pressure sensor 48 may be pneumatically coupled to the pressure port 49. The pressure port 49 may be arranged such that the air flow F1 passes beneath the pressure port 49 immediately before it enters the inhaler housing 20 at the air inlet. In some examples, the electronics module 40 does not have any structure that projects into the air flow F1. In such examples, it is ensured that the air flow F1 is disturbed as little as possible by the presence of the electronics module 40. Further, in some examples, the pressure sensor 48 and the pressure port 49 may be located external to the airflow channel F1 and F1' through which air and the medicament is drawn by the user during inhalation.

In use, the air flow F1 caused by inhalation by the patient causes a negative pressure at the pressure port 49 due to the Bernoulli/Venturi effect caused by the acceleration of the air flow when it enters the inhaler 210. The magnitude of the negative pressure is directly related to the magnitude of the flow rate of the air flow F1 and the relative distance between the pressure sensor 48 (*e.g.,* the pressure port 49) and the open upper end 22 of the housing 20. The negative pressure is registered by the pressure sensor 48. The processor 42 receives the pressure measurements from the pressure sensor 48, and may determine one or more inhalation parameters, such as flow rate.

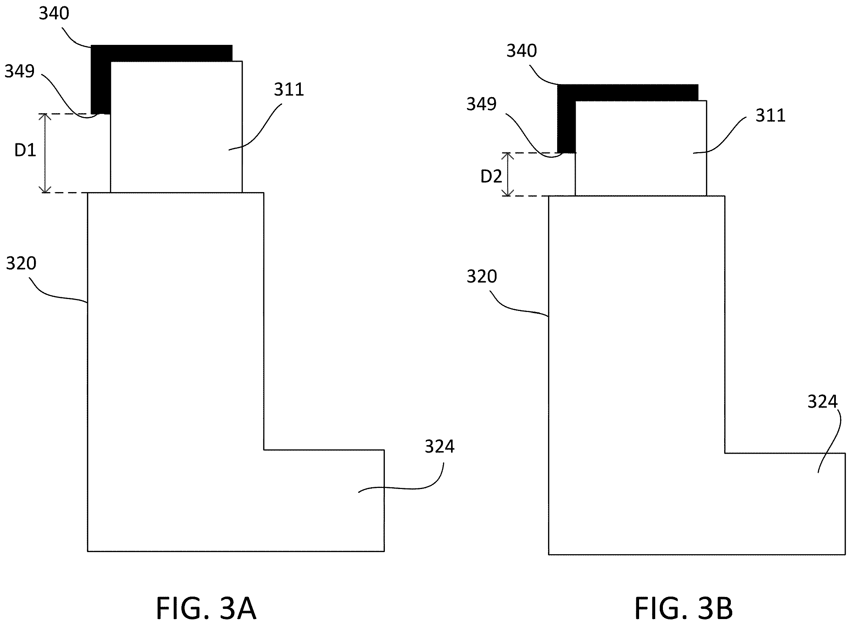

When the canister 11 is actuated, the position of the pressure sensor 48 (*e.g.,* and the pressure port 49) relative to the airflow channel of the inhaler 210 and the mouthpiece 24 of the inhaler 210 may change. Fig. 3A is an example block diagram that illustrates the location of an electronic module 340 before a canister 311 of an inhaler 320 is actuated (*e.g.,* depressed) to dispense a dose of medicament. Fig. 3B is an example block diagram that illustrates the location of the electronic module 340 when the canister 311 of the inhaler 320 is actuated (*e.g.,* depressed) to dispense a dose of medicament. The electronics module 340 may be an example of the electronics module 40, the canister 311 may be an example of the canister 11, and the inhaler 320 may be an example of the inhaler 210 of Fig. 2.

The canister 311 is in a non-actuated position in Fig. 3A. When the canister 311 is in the non-actuated position (*e.g.,* an at rest position), a pressure port 349 (*e.g.,* and a pressure sensor of the electronics module 340) is located at a distance D1 from an open upper end of the housing of the inhaler 320 (*e.g.,* and/or from the flow channel). When the canister 311 is actuated to dispense a dose of medication, as shown in Fig. 3B, the location of the electronics module 340 relative to the inhaler housing and/or the flow channel changes. For example, in Fig. 3B, the pressure port 349 (*e.g.,* and a pressure sensor of the electronics module 340) is located at a distance D2 from the open upper end of the housing of the inhaler 320 (*e.g.,* and/or from the flow channel) when the canister 311 is moved into the actuated position (*e.g.,* to dispense medicament to the user). The distance D2 is less than the distance D1.

Referring back to Fig. 2, since the distance between the pressure sensor 48 (*e.g*., and/or the pressure port 49) and the flow channel changes when the canister 11 is moved from the non-actuated position to the actuated position, the pressure measurements received by the processor 42 from the pressure sensor 48 also change. For example, this is at least in part because the pressure measurements calculated by the pressure sensor 48 are directly related to the magnitude of the flow rate of the air flow F1 through the airflow channel of the inhaler and the relative distance between the pressure sensor 48 (*e.g.,* the pressure port 49) and the open upper end 22 of the housing 20. So the change in the distance between the pressure sensor 48 and the open upper end 22 of the housing 20 (*e.g.,* and/or from the flow channel) causes a distinguishable change in pressure measurements detected by the pressure sensor 48. For example, the change in distance results in a change is resistivity between the pressure port 49 of the pressure sensor 48 and the flow channel. For example, the resistance between the pressure port 49 and the flow channel may reduce when the distance reduces, which may cause a distinguishable change in pressure measurements detected by the pressure sensor 48.

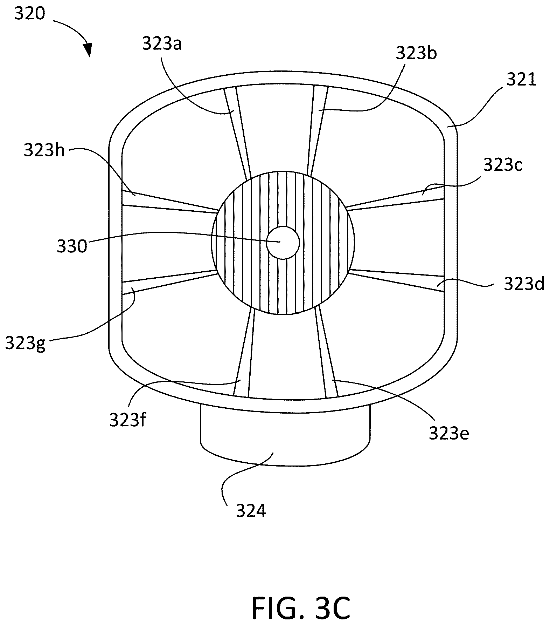

FIG. 3C is an example block diagram that illustrates a top down perspective of the inhaler 320 with the canister 311 removed. The inhaler 320 may define a housing that, for example, may be boot shaped as illustrated (*e.g.,* although other housing shapes are considered within the scope of the application). The housing may be configured to receive a medication canister, such as the canister 311. The interior of the inhaler 320 may be defined by a circumferential housing wall 321. At an open upper end of the receiving portion, the housing wall 321 may include a plurality of spacer ribs 323a-h that extend radially inwardly from the housing wall 321 (*e.g.,* similar to the spacer ribs 23 of the inhaler 210). As such, the outer circumferential wall of the canister may be spaced from the housing wall 321 of the inhaler 320, and axially extending air channels are thus formed between the housing wall 321 and the container when the container is installed within the inhaler 320. These air channels form the airflow channel of the inhaler 320.

At the lower end within the inhaler 320, the inhaler 320 may include a hollow socket 330 (*e.g.,* similar to the hollow socket 30 of the inhaler 210). A valve stem of the drug container may be seated in the hollow socket 330 when the container is installed within the inhaler 320. The hollow socket 330 may define a duct leading from the exit of the valve stem to a nozzle orifice. Finally, the inhaler 320 may include a mouthpiece 324.

The electronics module 340 may be located such that a pressure port *(e.g.,* the pressure port 49) may be aligned between any of the spacer ribs 323a-h. In some examples, the pressure port of the electronics module 340 may be aligned at a position A that is located between with the spacer ribs 323a and 323b. When aligned at the position A, the pressure port may be configured such that is aligned with an opening between the medication canister and the housing wall 321 (*e.g.,* aligned with the air channel that is located as position A). However, in other examples, the pressure port of the electronics module may be aligned at a different, radial position along the housing wall 321. For example, the pressure port may be aligned at a position B that is located between the spacer ribs 323a and 323h, a position C1 that is located between the spacer ribs 323g and 323f, a position D that is located between the spacer ribs 323e and 323d, or a position F that is located between the spacer ribs 323c and 323b. Further, although described as including eight spacer ribs 323, the inhaler 320 may include more or less spacer ribs, and the spacer ribs may be located in different radial positions along the housing wall 321. As such, the pressure port of the electronics module 340 may be located in a position other than those described herein.

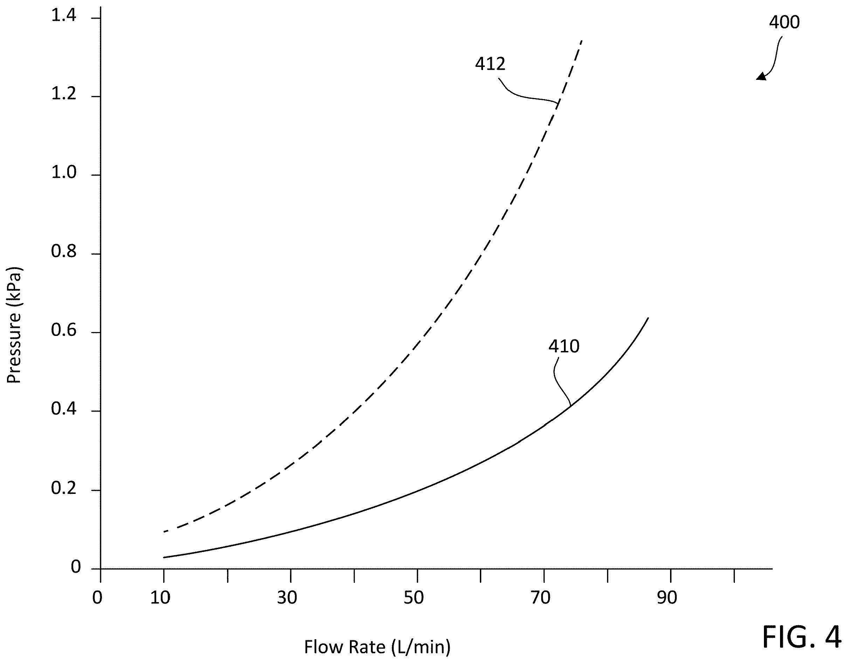

Fig. 4 is a graph of two example airflow rate versus pressure profiles. The airflow rates and profiles shown in Fig. 4 are merely examples and the determined rates may depend on the size, shape, and design of the inhaler and its components. The profile 410 illustrates an example of the measured pressure at each flow rate through the airflow channel of an inhaler (*e.g.,* such as the inhaler 210) when an electronics module (*e.g.,* and a pressure sensor and pressure port) is located at a first distance from the airflow channel, such as when the canister in an unactuated position (*e.g.,* the pressure port is located at the distance D1 from the open upper end of the inhaler housing). The profile 412 illustrates an example of the measured pressure at each flow rate through the airflow channel of the inhaler when the electronics module (*e.g.,* and the pressure sensor and pressure port) is located at a second distance from the airflow channel, such as when the canister in actuated to dispense a dose of medication (*e.g.,* the pressure port is located at the distance D2 from the open upper end of the inhaler housing). As noted, for the same flow rate through the inhaler, the measured pressure is greater when the distance is reduced, such as when the canister is moved from the non-actuated position (*e.g.,* D1) to the actuated position (*e.g*., D2) (*e.g*., due to a reduction is resistance between the pressure port and the flow channel).

Referring back to Fig. 2, the processor 42 may be configured to detect this sudden change in pressure and determine that it was the result of the canister 11 being actuated to dispense a dose of medicament to the user. As such, the processor 42 may be configured to determine the position of the pressure sensor 48 and/or the medication canister 11 of the inhaler 210 while the user inhales through the inhaler 210. Further, the processor 42 may be configured to detect whether an inhalation by a user through the inhaler 210 occurs prior the canister 11 of the inhaler 210 being depressed to release medication based on pressure measurements received from the pressure sensor 48. The processor 42 may be configured to determine the relative timing of a user's inhalation and the actuation of the canister using the pressure measurements (*e.g.,* only the pressure measurements) received from the pressure sensor.

Fig. 5A is a diagram of example pressure measurements 500 received by an electronics module of an inhaler (*e.g.,* the electronics module 40 of the inhaler 210), for example, when the user stops inhaling at the same time that they release a medicament canister of the inhaler. Fig. 5B is a diagram of example pressure measurements 520 calculated by an electronics module of an inhaler (*e.g.,* the electronics module 40 of the inhaler 210), for example, when the user releases the medicament canister of the inhaler before they stop inhaling through the mouthpiece. Referring to Fig. 5A, the user may begin inhaling through the inhaler at 502. When the user begins to inhale through the mouthpiece of the inhaler, the electronics module may receive pressure measurements from a pressure sensor (*e.g.,* and, in some example, may calculate a flow rate based on the received pressure measurements). The electronics module may generate an inhalation event when the pressure measurements (*e.g.,* and/or the calculated flow rate) exceed a threshold. As noted, the threshold may be an absolute change in pressure (*e.g.,* 10 kPa) and/or a rate of change threshold (*e.g.,* a slope threshold).

After the user begins inhaling, the user may actuate the canister at 504, for example, through the application of a force to the top of the canister or through the force of their inhalation, in the case of a pMDI. When the canister actuates, the position of the pressure sensor relative to the flow channel of the inhaler may change (*e.g.,* such as the change from the distance D1 to the distance D2 illustrated in Fig. 3A-B). The change in the position of the pressure sensor relative to the flow channel may cause the electronics module to receive pressure measurements that are distinguishably different from the pressure measurements received prior to the actuation of the canister. The electronics module may determine that the canister is actuated (*e.g.,* and/or that the canister and/or pressure sensor is moved into a second position) based on the distinguishable change in pressure measurements and/or calculated flow rates at 504. The example flow diagram 500 of Fig. 5A illustrates this distinguishable change as a step at 504, although the degree of this change may be different based on differently designed inhalers. In some examples, the electronics module may determine this canister actuation event based on a slope (*e.g.,* a rate of change) in the received pressure measurements and/or calculated flow rates that exceed a canister actuation threshold (*e.g.,* which may be a pressure threshold, a flow rate threshold, and/or a slope threshold (*e.g.,* a change in pressure or flow rate over time)). In response, the electronics module may record a canister actuation event.

The user may continue to inhale through the mouthpiece of the inhaler after actuating the canister at 504. As such, the received pressure measurements and/or calculated flow rates may continue to increase (*e.g.,* increase in the negative direction). At some point, the user's inhalation will begin to slow down, such as is represented by 506 in the flow diagram 500. Then, at 508, the user may stop inhaling and may release the canister, which will result in the received pressure measurements and/or the calculated flow rates returning to their original value prior to the user's interaction, which in this example is 0 LPMs. In examples that incorporate a barometric pressure sensor, the processor may be configured to zero out an average of the barometric pressure prior to inhalation to determine the resultant change in pressure that is due to the user's inhalation and the canister actuation.

The pressure measurement profile 520 of Fig. 5B is similar to the pressure measurement profile 500 of Fig. 5A, with the exception that the user releases the canister while the user is still inhaling through the mouthpiece of the inhaler. For example, the user may begin their inhalation at 522 and actuate the canister to release a dose of medicament at 524. The electronics module may determine that the canister is actuated (*e.g.,* and/or that the canister and/or pressure sensor is moved into a second position) based on the distinguishable change in pressure measurements and/or calculated flow rates at 524 (*e.g.,* by comparing the pressure measurements to a canister actuation threshold, which may be a slope threshold or an absolute pressure measurement value). Then, at 526 while the user is still inhaling through the inhaler, the user may release the canister and the canister and pressure sensor may move back to the original position relative to the flow channel. As such, the electronics module may determine that the canister has returned to the un-actuated position (*e.g.,* and/or that the canister and/or pressure sensor has returned to the first position) based on the distinguishable change in pressure measurements and/or calculated flow rates at 526. Finally, the user's inhalation may slow before the inhalation stops at 528. In this example, the electronics module may determine when the user actuated the canister (at 524) and also when the user released the canister (at 526) due to there being two distinguishing pressure changes in the flow profile.

Referring back to Fig. 2, the processor 42 may be configured to determine the position of the pressure sensor 48 and/or the medication canister 11 of the inhaler 210 while the user inhales through the inhaler 210 based on the received pressure measurement while the user is inhaling through the inhaler. Further, the processor 42 may be configured to detect whether an inhalation by a user through the inhaler 210 occurs prior the canister 11 of the inhaler 210 being depressed to release medication based on pressure measurements received from the pressure sensor 48. With MDIs, for example, the user tends to receive a greater, and proper dose of medicament when the user inhales prior to actuating the canister. Existing inhalers may attempt to determine the timing of the user inhalation relative to the canister actuation, but these inhalers use multiple distinct sensors for detecting inhalation and for detecting canister actuation, which can result in less accurate feedback and increased production costs. The electronics module 40 is configured to determine that a user is inhaling and how well, and also determine when the canister 11 is actuated, through the use of one pressure sensor.

The pressure sensor 48 and/or the pressure port 49 may be attached to or moveable in relation to any movement of the canister 11. As such, when the canister 11 is depressed either by the user's actuation or inhalation (*e.g.,* in the case of a breath actuated MDI), the pressure sensor 48 and/or pressure port 49 is configured to move relative to the flow channel of the inhaler 210. Accordingly, (*e.g.,* while a user is inhaling through the inhaler 210) the pressure sensor 48 may be configured to measure a first pressure when the pressure sensor 48 is in the first position (*e.g.,* prior to the actuation of the canister 11) relative to the flow channel of the inhaler 210, and a distinguishable second pressure when the pressure sensor 48 is in the second position (*e.g.,* a depressed position to release a dose of medication) relative to the flow channel of the inhaler 210.

While the user is inhaling through the inhaler 210, the pressure measurements may change by a distinguishable amount (*e.g.,* a step, such as shown in the examples of FIG. 5A and 5B) when the position of the pressure sensor 48 relative to the flow channel changes upon actuation of the canister 11. The processor 42 may be configured to detect whether an inhalation by a user through the inhaler 210 occurs prior to the canister 11 being depressed to release medication based on a distinguishable change is pressure measurements received from the pressure sensor 48. As noted above, this distinguishable second pressure may be detected by determining that a slope of the received pressure measurements (*e.g.,* and/or calculated flow rates) exceeds a slope threshold that is indicative of the canister actuation. Further, as noted above, the processor 48 may be configured to determine the position of the pressure sensor 48 and/or the canister 11 (*e.g.,* in the first position or the second position) based on the received pressure measurements.

In some examples, the pressure sensor 48 is configured such that it does not extend into the inhaler housing 20 when the pressure sensor 48 is in the first position and/or the second position. For example, the pressure sensor 48 may be configured such that a gap exists between the pressure sensor 48 (*e.g.,* and pressure port 49) and the airflow channel and/or the open upper end 22 of the inhaler 210 when the electronics module 40 and/or the canister 11 are in the first position and/or the second position. As noted herein, the pressure port 49 may be aligned with an opening between the canister 11 and the housing 20 *(e.g.,* the open upper end 22) when the electronics module 40 is connected to the canister 11. However, in some examples, the pressure port 49 does not extend into or between the housing 20 and the canister 11. Accordingly, in some examples, the pressure sensor 48 is pneumatically coupled to the airflow channel, but the pneumatic coupling is rather weak (*e.g.,* due to the gap between the sensor port and the inhaler housing). Since, in some examples, the pneumatic coupling between the pressure sensor 48 and the flow channel of the inhaler 210 is rather weak, the pressure measurements may change by a distinguishable amount when the position of the pressure sensor 48 and/or pressure port 49 relative to the flow channel changes upon actuation of the canister 11.

The processor 42 and/or the pressure sensor 48 may be configured with a first flow rate calibration curve (*e.g.,* the profile 410 from Fig. 4) for when the pressure sensor is in the first position, and a second flow rate calibration curve (*e.g.,* the profile 412 from Fig. 4) for when the pressure sensor is in the second position. Further, in some examples, the processor 42 and/or the pressure sensor 48 is calibrated based on only one of the first position or the second position (*e.g.,* the profile 410 or the profile 412), which for example, may ensure the distinguishable change in pressure measurements when the pressure sensor 48 changes position relative to the flow channel based on the actuation of the canister 11.

The electronics module 40 may turn on the pressure sensor 48 and/or begin receiving measurements from the pressure sensor 48 in response to switching from a low power state, such as an off state or a sleep state, to an on state. The processor 42 may calculate a flow profile using the received pressure measurements. The processor 42 may compare the measurements received from the pressure sensor 48 (e.g., the flow profile) to one or more thresholds and determine whether the measurements are indicative of an inhalation by the user. For example, the processor 42 may be configured to compare the pressure measurements to an inhalation threshold, and determine (*e.g.,* generate) an inhalation event based on the flow rates received from the pressure sensor 48 exceeding the inhalation threshold. The processor 42 may be configured to compare the pressure measurements to a canister actuation threshold, and determine (*e.g.,* generate) a canister actuation event based on the flow rates exceeding the canister actuation threshold. The canister actuation threshold may be larger than the inhalation event *(e.g.,* a larger negative flow rate), for example since the actuation of the canister 11 may bring the pressure sensor 48 closer to the flow channel.

The processor 42 may be configured to compare the pressure measurements to a canister released threshold, and detect the movement of the canister back to the first position based on the flow rates falling below the canister released threshold. The processor 42 may be configured to determine a duration of an inhalation event based on the amount of time that the pressure measurements (*e.g.,* flow rates) exceed the inhalation threshold. Alternatively, the duration of the inhalation event may start at the time of the canister actuation event and ends when the flow rates fall below the inhalation threshold. Although described with reference to pressure measurements, in some examples, the processor 42 may be configured to generate one or more inhalation parameters (*e.g.,* such as flow rates) based on the received pressure measurements, and compare the inhalation parameters to one or more thresholds to determine one or more events.

The processor 42 may be configured to generate one or more error events based on feedback from the pressure sensor 48. For example, the processor 42 may be configured to generate a late inhalation error event when the detection of an inhalation occurs after the medication canister 11 is depressed. For example, the processor 42 may be configured to detect an inhalation event based on the pressure measurements received from the pressure sensor 48 exceeding an inhalation threshold (*e.g.,* a pressure threshold and/or a flow rate threshold), and detect a canister actuation event based on the pressure measurements exceeding a canister actuation threshold *(e.g.,* a pressure threshold and/or a flow rate threshold). The processor 42 may be configured to generate the late inhalation error event when the inhalation event occurs after the canister actuation event *(e.g.,* or occurs before the canister actuation event, but not sufficiently far in advance of the canister actuation event). For example, the processor 42 may detect a user's inhalation and detect the canister 11 being released (unactuated) during the user's inhalation. In such instances, the processor 42 may generate a late inhalation event. Further, in some instances, the processor 42 may not detect a canister actuation or release (*e.g.,* because the canister 11 was actuated prior to the user's inhalation started and released after the inhalation ended, or the canister wasn't actuated at all). In response, the processor 42 may generate a late inhalation error event.

In some examples, the processor 42 may be configured to send, via the communication circuit 46, the late inhalation error event to the external device 60 for display via a user interface of the external device 60. The late inhalation error event may include a notification that the user actuated the canister too early / inhaled too late and/or may indicate the relative timing of the user's inhalation and the canister actuation and/or release. Further, in some instance, the late inhalation error event may indicate that the processor 42 could not determine whether the canister 11 was actuated before the inhalation began or whether the canister 11 was simply not actuated at all (*e.g.,* when the processor 42 does not detect the canister actuation or release during the user's inhalation).

The processor 42 may be configured to generate a late actuation error event when the pressure measurements (*e.g.,* flow rates) exceed a threshold for a period of time that exceeds a duration threshold prior to the detection of the canister 11 being actuated. For example, the processor 42 may determine that the user inhales for too long of a period of time prior to the canister 11 being actuated, and in response, generate the late actuation error event (*e.g.,* an early inhalation error event). In some examples, the processor 42 may be configured to determine that the pressure measurements resulted in a PIF that exceeded a threshold (*e.g.,* 30 LPM or 45 LPM) prior to the canister being actuated, and in response, generate the late actuation error event. The processor 42 may be configured to send, via the communication circuit 46, the late actuation error event to the external device 60 for display via a user interface of the external device 60. The late actuation error event may include a notification that the user actuated the canister too late (*e.g.,* or that the user inhaled too early) and/or may indicate the relative timing of the user's inhalation and the canister actuation.

The processor 42 may be configured to generate an on-time inhalation event if the user's inhalation occurred within a predetermined time period before the canister was actuated (*e.g.,* within 300-800 milliseconds before canister actuation). Further, the processor 42 may generate the on-time inhalation event if the user's inhalation occurred the predetermined time period before the canister was actuated and if it reach a particular threshold value (*e.g.,* a PIF value, such as a PIF of at least 10 or 15 LPM) before the canister was actuated (*e.g.,* and in some instances, did not reach an upper threshold value, such as 40 LPM prior to canister actuation), but did not trigger a late actuation error event.

The processor 42 may be configured to send the events, via communication circuit 46, to the external device 60 for display via a user interface of the external device 60 (*e.g.,* a smartphone, tablet, PC, etc.). Alternatively or additionally, the processor 42 may send the pressure measurements and/or calculated inhalation parameters to the external device 60, and the external device 60 may determine any combination of the events described herein, for example, prior to displaying an indication of the events via the user interface. Further, the processor 42 and/or the external device 60 may timestamp and/or geotag the events and/or usage parameters (*e.g*., using a real-time stamp or a relative time stamp that begins when the user first operates the inhaler.

In some examples, the processor 42 may include a mechanical switch configured to be actuated prior to, during, or after use of the inhaler 210. The mechanical switch may indicate that a dose of medicament has been primed and is ready for inhalation (e.g., such as by metering a dose from a hopper, advancing and/or opening a blister pack, breaking open and/or spinning a pill comprising medicament, the inhaler has been shaken, etc.).

In some examples, the inhaler may be a dry powder inhaler that comprises dry powder medicament. The inhaler 210 may include a medicament reservoir (that is different from the canister 11) that houses medicament (*e.g.,* a blister strip, a hopper, *etc*.), and/or a dose metering assembly configured to meter a dose of the medicament from the reservoir. The dose metering assembly may include a bellows that is configured to meter a dose of dry powder medicament from a hopper, a blister strip advancement and opening mechanism, a chamber configured to open a pill of dry powder medicament, and/or the like. The processor 42 may be configured to register the metering of the dose by the dose metering assembly, each metering being thereby indicative of the inhalation performed by the user using the inhaler 210. Accordingly, the inhaler 210 may be configured to monitor the number of inhalations of the medicament, since the dose should be metered via the dose metering assembly before being inhaled by the user. Alternatively or additionally, the processor 42 may be configured to register a usage event based on the metering of the dose by the dose metering assembly and register another usage event based on feedback from one or more sensors. In such examples, the processor 42 may be configured to correlate the timing and/or duration of each of the usage events, which for example, may be used to determine whether the user proper primed the inhaler 210 and properly inhaled the dose of medicament, since both steps are valuable in determining whether a successful dose is received.

The electronics module 40 may include one or more of a switch configured to detect usage of inhaler 210, one or more sensors configured to detect use of inhaler 210 (*e.g.,* one or more sensors in addition to the pressure sensor 48), one or more buttons configured to be depressed upon use of inhaler 210, and/or the like. As such, a usage parameter may be one or more measurements performed by the electronics module 40, such as a count of the number of uses inhaler 210, a measure of airflow of inhaler 210, a detection of the canister 11 being actuated, and/or another measurement indicating the usage of the medicament of inhaler 210. The electronics module 40 may register each inhalation in different manners and/or based on additional or alternative feedback. For example, the electronics module 40 may be configured to register an inhalation by the user when the feedback from a suitable sensor indicates that an inhalation by the user has occurred, for example when a pressure measurement or flow rate exceeds a predefined threshold associated with a successful inhalation, when a medication canister of the inhaler is actuated, *etc.*

The electronics module 40 includes a housing, which in some examples, may be fastened to the canister 11 through fastening means (not shown). The fastening structure may include any combination of elements, such as a magnet, glue, Velcro^{®}, a cavity formed in a housing of the electronics module 40 and configured to fit around a portion of the canister 11 and/or inhaler 210 in a press fit, a strap or band configured to be tied to secure the housing of the electronics module 40 to the canister 11 and/or inhaler 210, a strap or band configured to elastically secure the housing of the electronics module 40 to the canister 11 and/or inhaler 210 similar to a rubber band, a clip configured to clip t the housing of the electronics module 40 to the canister 11 and/or inhaler 210, and/or a guide track configured to slidably receive a portion of the inhaler 210 therein. The electronics module 40 may be fastened/attached to the canister 11 in a defined axial position. In some examples, the electronics module 40 may be configured to determine the axial position that it is installed based on received pressure measurements (*e.g.,* during an actuation and inhalation by the user).

The electronics module 40 may include an indicator, such as a light, speaker, a display, a vibrating element, etc., that is configured to generate user feedback for the user. For instance, the indicator may be configured to generate an audible signal that indicates to the user whether the flow rate is within a desired range, for example, after inhalation but prior to canister actuation. As another example, the indicator may generate a tactile signal in the form of a vibration pattern that indicates to the user whether the flow rate is within a desired range, for example, after inhalation but prior to canister actuation. As yet another example, the indicator may generate a visual signal that indicates to the patient whether the flow rate is within a desired range, for example, after inhalation but prior to canister actuation.

Further, as previously noted, the timing between the user inhalation and canister actuation not only affects the amount of medicament that the user receives, but is also affects how deep in the lungs the medicament is delivered. Some active pharmaceutical ingredients are used to target specific portions of the user's respiratory track, and as such, it is desirable to deliver a maximum amount of each dose to that targeted portion of the user's respiratory track. For example, some medications may target the upper airways/respiratory track of the user, while others might target the lower airways/respiratory track of the user. In such instances, the processor 42 of the electronics module 40 may be configured to generate the canister actuation notification during the user's inhalation (*e.g.,* based on inhaled volume and/or duration) to alert the user to actuate the canister to deliver a dose of medicament at a time that will increase the amount of the dose that is delivered to the targeted portion of the user's respiratory track. The processor 42 may generate the canister actuation notification using the indicator of the electronics module 40 (*e.g.,* the processor 42 may illuminate a light of the electronics module 40, cause a speaker of the electronics module 40 to output an audible sound, generate a tactile signal in the form of a vibration pattern using a vibrating element, *etc.).*

For instance, the processor 42 may receive pressure measurements from the pressure sensor 48 during a user's inhalation. As noted above, the processor 42 may determine one or more inhalation parameters based on the pressure measurements, such as inhaled volume and/or inhalation duration. The processor 42 may generate the canister actuation notification when the inhalation parameter reaches a particular threshold *(e.g.,* a canister actuation notification threshold), where the threshold may be dependent on the portion of the user's respiratory track that is targeted by the medicament of the inhaler. When the inhalation parameter is inhaled volume, the canister actuation notification threshold may represent a volume of the user's inhalation. When the inhalation parameter is inhalation duration, the canister actuation notification threshold may represent a duration of the user's inhalation. For example, when the medicament is used to target the upper airways, the threshold may be a larger value of the inhalation parameter (*e.g.,* a larger inhaled volume or larger/longer inhalation duration), such that the processor 42 is configured to generate the notification later during the inhalation (*e.g.,* to ensure that a greater portion of the medication is provided to the upper airways, and less is delivered to the lower airways of the user). Conversely, when the medicament is used to target the lower airways, the threshold may be a smaller value of the inhalation parameter (*e.g.,* a smaller inhaled volume or smaller/shorter inhalation duration), as compared to when the medicament is used to target the upper airways. As such, the processor 42 may be configured to generate the notification earlier during the user's inhalation, for example, to ensure that a greater portion of the medication is provided to the lower airways, and less is delivered to the upper airways of the user.

Further, the processor 42 may be configured to generate one or more notifications based on a comparison of the generation of the canister actuation notification and the detection of the actuation of the canister 11. For example, the processor 42 may be configured to send data to the external device 60, where the data indicates the relative timing between the generation of the canister actuation notification and the detection of the actuation of the medication canister 11. The external device 60 may receive the data and cause a display (*e.g.,* a display of the external device or of a different device) to present a notification to the user that indicates the relative timing between the generation of the canister actuation notification and the detection of the actuation of the medication canister 11. In some examples, the notification may indicate that the user did not actuate the canister in a timely manner after the generations of the canister actuation notification. This may assist the user by informing them as to their relative coordination between the generation of the canister actuation notification and the processors 42 detection of the actuation of the medication canister 11.

As noted above, the processor 42 may be configured to detect a user's exhalation through the inhaler 20. Further, the processor 42 may be configured to detect the duration of time that a user holds their breath based on measurements received from the pressure sensor 48. For example, the processor 42 may detect the beginning of the user's inhalation when the received pressure measurements exceed an inhalation threshold (*e.g.,* a slope in pressure measurements that is indicative of a user's inhalation), and detect when the user stops inhaling (*e.g.,* has inhaled completely) based on the received pressure measurements (*e.g.,* when the pressure measurements return to or fall below a particular value). The processor 42 may detect when the user exhales their breath based on received pressure measurement (*e.g.,* based on the user exhaling through the inhaler 20). The processor 42 may determine the time period that the user held their breath based on the time difference between the end of the user's inhalation (*e.g.,* or, in some examples, the start of the user's inhalation) and the detection of the user's exhalation. The time that the user's held their breath can be indicative of the amount of medicament that the user receives during their inhalation, as typically, a longer breath hold results in more medicament being delivered into the user's respiratory system.

Further, the processor 42 may be configured to generate one or more notifications based on the time that the user held their inhalation. For example, the processor 42 may be configured to send data to the external device 60, where the data indicates the time that the user held their inhalation. The external device 60 may receive the data and cause a display (*e.g.,* a display of the external device or of a different device) to present a notification to the user that indicates the time that the user held their inhalation. In some examples, the notification may indicate that the user did not hold their inhalation for a long enough time to ensure a proper dose of medicament was received. Alternatively or additionally, the processor 42 may cause one or more indicators of the electronics module 40 to change states when the processor 42 determines that the user has held their inhalation for a predetermined amount of time (*e.g.,* which may be adjustable based on medication type and target region of the respiratory track), for example, to indicate that the user may stop holding their inhalation and exhale (*e.g.,* by turning a light from on to off, changing the color state of a light, generating an audible output, etc.).

The electronics module 40 may use feedback from a sensor, such as an accelerometer and/or touch sensitive device to change power states. For example, the electronics module 40 may reside in a low power state, such as an off state or a sleep state, when not in use. During the low power state, any combination of sensors, the communication circuit 46, and/or various pins on the processor 42 may be off (*e.g.,* other than the sensor and associated pin on the processor that is used to trigger the electronics module 40 to switch power states). The electronics module 40 may switch from the low power state to an on state in response to receiving feedback from a sensor, such as feedback from an accelerometer that indicates that the user is shaking the inhaler 210 (*e.g.,* an MDI inhaler) or feedback from a touch sensitive device that indicates that the user has the inhaler 210 in their hand. The electronics module 40 may turn on the communication circuit 46 and/or the pressure sensor 48, and/or begin receiving measurements from the pressure sensor 48 in response to switching from a low power state, such as an off state or a sleep state, to an on state.

The external device 60 may include a smart phone (e.g., an iPhone^{®} smart phone, an Android^{®} smart phone, or a Blackberry^{®} smart phone), a personal computer, a laptop, a wireless-capable media device (e.g., MP3 player, gaming device, television, a media streaming devices (e.g., the Amazon Fire TV, Nexus Player, *etc*.), a tablet device (e.g., an iPad^{®} hand-held computing device), a Wi-Fi or wireless-communication-capable television, a hub device or smart speaker (*e.g.,* Amazon Echo, Google Home, *etc*.), or any other suitable Internet-Protocol-enabled device.

The external device 60 may be configured to execute a computer program (*e.g.,* a mobile application) that causes the external device 60 to receive one or more signals from the electronics module 40 via the wireless link and to generate user feedback using an output device of the external device 60. For instance, the external device 60 may be configured to display user feedback and/or instructions for indicating the correct operation of the inhaler 210 on a display screen of the external device 60. The external device 60 may output an audible signal via a loudspeaker of the remote device that indicates the correct operation of the inhaler 210. The external device 60 may be configured to provide tactile feedback (*e.g.,* by vibrating) depending on the manner in which the patient handles the inhaler. This can all be performed in real time with an inhalation by the user through the inhaler 210. The external device 60 may be configured to store received signals for later readout, and/or to transmit the received signals or data derived from the received signals to a remote server for analysis. This enables the monitoring of the usage of the inhaler 210 by medical personnel. In other embodiments, the inhaler 210 may be configured to communicate directly with a remote server, for example, when the electronics module 40 includes a cellular chipset.

The external device 60 may include a communication circuit, and as such may be configured to transmit and/or receive RF signals via a Wi-Fi communication link, a Wi-MAX communications link, a Bluetooth^{®} or Bluetooth Smart communications link, a near field communication (NFC) link, a cellular communications link, a television white space (TVWS) communication link, or any combination thereof. As further described herein, the external device 60 may transfer data (*e.g.,* through the public and/or private network) to a remote server using, for example, a communication interface published by the remote server, such as a dedicated API. For example, the external device 60 may send usage data relating to one or more paired inhalers 210 to the remote server.

The external device 60 may comprise any combination of a processor, memory, and/or software configured to perform the functions described herein for the external device 60. For example, the processor may be a general purpose processor programmed with computer executable instructions for implementing the functions of the external device 60. The processor may be implemented using a microprocessor or microcontroller configured to perform the functions of the external device 60. The processor may be implemented using an embedded processor or digital signal processor configured to perform the functions of the external device 60.

The memory of external device 60may comprise a computer-readable storage media or machine-readable storage media that maintains computer-executable instructions for performing one or more as described herein. For example, the memory may comprise computer-executable instructions or machine-readable instructions that include one or more portions of the procedures described herein. The processor of the external device 60 may access the instructions from memory for being executed to cause the processor to operate as described herein, or to operate one or more other devices as described herein. The memory may comprise computer-executable instructions for executing configuration software. For example, the computer-executable instructions may be executed to perform at least a portion of the procedure 800 as described herein. Further, the memory may have stored thereon one or more settings and/or control parameters associated with the external device 60.

The external device 60 may include a display device and software (e.g., a mobile application) for visually presenting or displaying the inhaler data associated with a given usage parameters and/or data related to usage events through a graphical user interface (GUI) on the display device. The external device 60 may also provide alerts or notification via the GUI and/or via the inhaler 210 by sending the inhaler 210 a message that causes the inhaler 210 to illuminate a light source of the inhaler 210 and/or generate an audible alert by way of a speaker of the inhaler 210.

The external device 60 may determine any of the events described herein, and display any of the events described herein. For example, in addition to or as an alternative to the electronics module 40 determining the one or more events, the external device 60 may determine any combination of the events described herein. The events may include, but are not limited to the inhalation event, the canister actuation event, the canister , the late inhalation error event, the late actuation error event, the shake event, the no inhalation event, low inhalations event, good inhalation event, excessive inhalation event, exhalation event, underuse event, overuse event, inhaler cap openings or medication priming event (*e.g.,* such as opening of a blister strip, the opening of a capsule, *etc.,* as described further herein), *etc.*

The external device 60 may receive the event from the inhaler 210, and may display, via the display device, an indication of the event. For example, the external device 60 may display an indication of the position of the canister and/or pressure sensor relative to the user's inhalation flow profile so the user and/or health care provider can assess whether the user is actuating the canister too early, too late, or on time. Further, and for example, the external device 60 may display a notification for the late inhalation error event that indicates that the user inhaled too late (or actuated the canister too early) and/or may indicate the relative timing of the user's inhalation and the canister actuation. Similarly, external device 60 may display a notification for the late actuation error event that indicates that the user inhaled too early (or actuated the canister too late) and/or may indicate the relative timing of the user's inhalation and the canister actuation.

Table 1 provides flow rates (in liters per minute (LPM)) that are calculated based on a pressure port of an electronics module (*e.g.,* the pressure port 49 of the electronics module 40) being aligned at various positions relative to the inhaler housing (*e.g.,* the housing of the inhaler 210) while a flow controller applied a 28.5 LPM simulated inhalation flow through the mouthpiece of the inhaler for 2.5 seconds. As noted above with respect to FIG. 3C, the pressure port of the electronics module may be aligned between two spacer ribs of the housing wall of the inhaler (*e.g.,* between the spacer ribs 23 of the inhaler 20). For instance, the pressure port may be aligned at the position A, the position B, the position C, the position D, or the position E as illustrated in the example shown in FIG. 3C. With the pressure port aligned at each of the positions A-E, the flow controller applied the 28.5 LPM simulated inhalation flow with the canister located in the first position (*e.g.,* prior to actuation, or in a non-actuated state) and in the second position (*e.g.,* with the canister actuated to dispense medication). The pressure measurements were captured by the pressure sensor of the electronics module, and provided to a processor for conversion to flow rates. Table 1 provides the resulting flow rates that are calculated when the pressure port is aligned at each of the positions A-E, and a flow controller applied a 28.5 LPM simulated inhalation flow through the mouthpiece of the inhaler for 2.5 seconds for each of the positions A-E.

**Table 1**

| **Position** | **First Position (LPM)** | **Second Position (LPM)** |
|---|---|---|
| A | 10.5 | 22 |
| B | 12.3 | 16.8 |
| C | 8.6 | 18.8 |
| D | 6.5 | 27.4 |
| E | 8.3 | 23.5 |

As shown in Table 1, the flow rates calculated when the canister was at the first position (e.g., prior to canister actuation) are distinguishably different from the flow rates calculated when the canister was at the second position (e.g., when the canister is actuated), for every position A-E. Accordingly, the electronics module and/or an external device that receives the pressure measurements may be configured to detect whether an inhalation occurs prior to the medication canister being depressed to release medication based on the pressure measurements, and/or determine the position of the pressure sensor and/or the medication canister based on the pressure measurements received from the pressure sensor while an inhalation is occurring through the mouthpiece of the inhaler.

Table 2 provides flow rates (in liters per minute (LPM)) that are calculated based on a pressure port of an electronics module (*e.g.,* the pressure port 49 of the electronics module 40) being aligned at various positions relative to the inhaler housing (*e.g*., the housing of the inhaler 210) while a flow controller applied a 60 LPM and 100 LMP simulated inhalation flow through the mouthpiece of the inhaler for 2.5 seconds. It should be appreciated that, even though the positions A-E around the inhaler housing are the same as was performed during the tests in Table 1, the position of the pressure sensor is not absolutely identical for both tests.

**Table 2**

| | **First Position (LPM)** | | | | | **Second Position (LPM)** | | | | |
|---|---|---|---|---|---|---|---|---|---|---|
| **Flow Rate (LPM)** | **A** | **B** | **C** | **D** | **E** | **A** | **B** | **C** | **D** | **E** |
| **60** | 20.32 | 19.26 | 13.22 | 11.31 | 7.08 | 34.69 | 31.31 | 17.10 | 20.46 | 17.72 |
| **100** | 34.53 | 24.36 | 33.53 | 17.43 | 15.73 | 72.99 | 51.94 | 40.63 | 32.06 | 30.37 |

As shown in Table 2, the flow rates calculated when the canister was at the first position (*e.g*., prior to canister actuation) are distinguishably different from the flow rates calculated when the canister was at the second position (*e.g*., when the canister is actuated), for every position A-E and for each of the simulated inhalation flows (*e.g.,* at rates of 60 and 100 LPM). Accordingly, the electronics module and/or an external device that receives the pressure measurements may be configured to detect whether an inhalation occurs prior to the medication canister being depressed to release medication based on the pressure measurements, and/or determine the position of the pressure sensor and/or the medication canister based on the pressure measurements received from the pressure sensor while an inhalation is occurring through the mouthpiece of the inhaler.

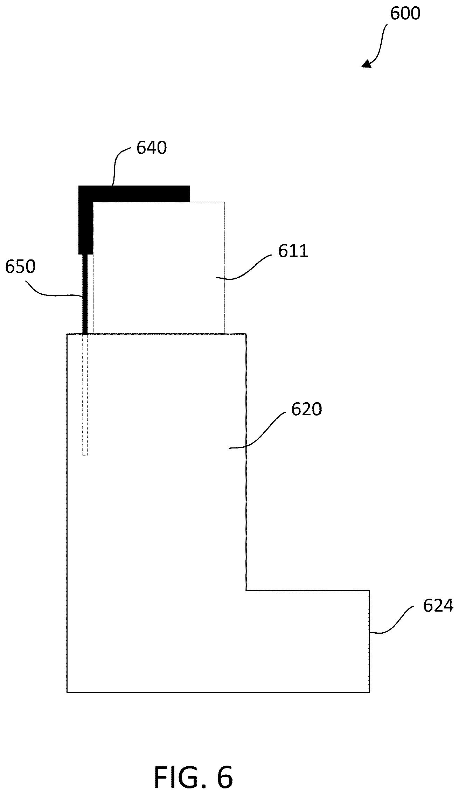

Fig. 6 is a diagram of an example inhaler 600 that includes an electronic module 640 that includes a capillary tube 650. The inhaler 600 includes a medicament container 611 and an inhaler housing 620. The medicament container 611 is an example of the medicament container 11, while the inhaler housing 620 is an example of the housing 20 of the inhaler 210. The electronics module 640 is an example of the electronics module 40 of the inhaler 210, but with the inclusion of the capillary tube 650. The capillary tube 650 extends from the electronics module 640 and into the inhaler housing 620. The capillary tube 650 moves from a first position relative to a mouthpiece 624 of the inhaler housing 620 when the canister 611 is in an un-actuated position to a second position relative to the mouthpiece 624 when the canister 611 is actuated. As such, a processor of the electronics module 640 is configured to detect the movement of the electronics module 640 and canister 611 relative to the mouthpiece 624 based on a distinguishable step in pressure measurements received from a pressure sensor of the electronics module 640 (*e.g.*, similar to the examples shown in Fig. 5A and Fig. 5B). Accordingly, the electronics module 640 may be configured to determine the position of the pressure sensor and/or the medication canister 611 of the inhaler 600 while the user inhales through the inhaler 600. Further, the electronics module 640 may be configured to detect whether an inhalation by a user through the inhaler 600 occurs prior the canister 611 of the inhaler 600 being depressed to release medication based on pressure measurements received from the pressure sensor.

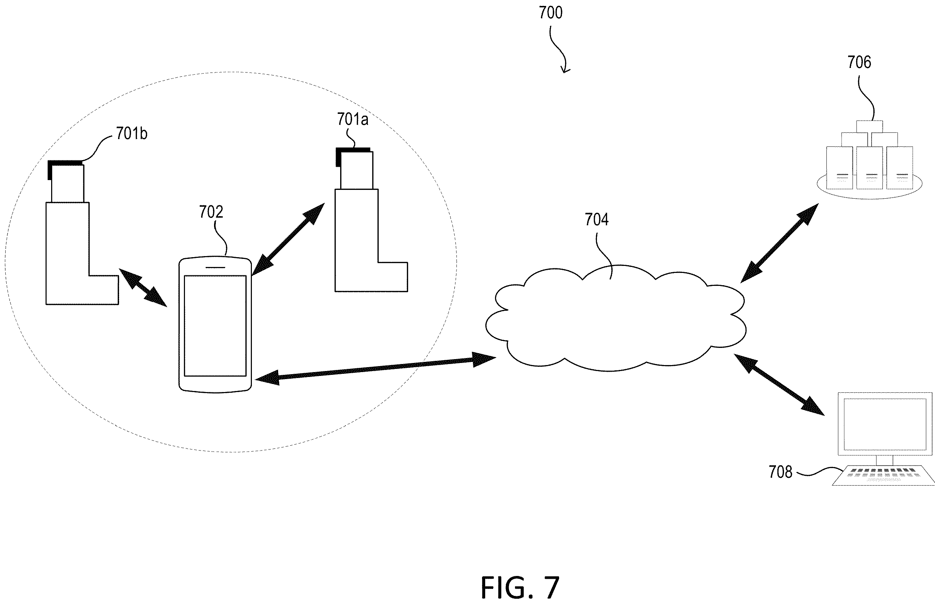

FIG. 7 is a diagram of an example system 700 including an inhalation device 701a, an inhalation device 701b, an external device 702 (e.g., a mobile device), a public and/or private network 704 (e.g., the Internet, a cloud network), a health care provider 706, and a third party computer or server 708 (e.g., friends, family, pharmaceutical manufacturer, etc.). The inhalation devices 701a, 701b may be examples of the inhaler 210 of Fig. 2. Accordingly, the inhalation devices 701a, 702b may include an electronics module, such as the electronics module 40 of the inhaler 210. Further, as noted herein, although described as being integrated as part of the inhaler, the electronics module of the inhalation devices 701a, 702b may be removeably and replaceably attachable and detachable from the inhalation devices 701a, 702b.

The mobile device 702 may be an example of the external device 40 from Fig. 2. The mobile device 702 may include a smart phone (e.g., an iPhone^{®} smart phone, an Android^{®} smart phone, or a Blackberry^{®} smart phone), a personal computer, a laptop, a wireless-capable media device (e.g., MP3 player, gaming device, television, a media streaming devices (e.g., the Amazon Fire TV, Nexus Player, etc.), etc.), a tablet device (e.g., an iPad^{®} hand-held computing device), a Wi-Fi or wireless-communication-capable television, or any other suitable Internet-Protocol-enabled device. For example, the mobile device 702 may be configured to transmit and/or receive RF signals via a Wi-Fi communication link, a Wi-MAX communications link, a Bluetooth^{®} or Bluetooth Smart communications link, a near field communication (NFC) link, a cellular communications link, a television white space (TVWS) communication link, or any combination thereof. The mobile device 902 may transfer data through the public and/or private network 704 to the health care provider 706 and/or one or more third party computers or serves 708 (e.g., friends, family, pharmaceutical company, etc.).

As noted above, the inhalation devices 701a, 702b may include a communication circuit, such as a Bluetooth radio, for transferring data to the mobile device 702. The data may include the measurement readings taken by the one or more sensors of the electronics modules of the inhalation devices 701a, 701b, parameters computed by the processor of the electronics modules, one or more events generated by the processor of the electronic modules, and/or other data captured by the inhalation devices 701a, 702b. The inhalation devices 701a, 702b may receive data from the mobile device 702, such as, for example, program instructions, operating system changes, dosage information, alerts or notifications, acknowledgments, etc.

The mobile device 702 may process and analyze the data to determine the usage parameters associated with the inhalation devices 701a, 702b. For example, the mobile device 702 may process the data to identify inhalation events, canister actuation events, error events, no inhalation events, low inhalations events, good inhalation events, excessive inhalation events and/or exhalation events. The mobile device 702 may also process the data to identify additional events. The mobile device 702 may further process the data to estimate the number of doses delivered and/or remaining and to identify error conditions, such as those associated with a timestamp error flag. The mobile device 702 may include a display and software for visually presenting the usage parameters through a GUI on the display.

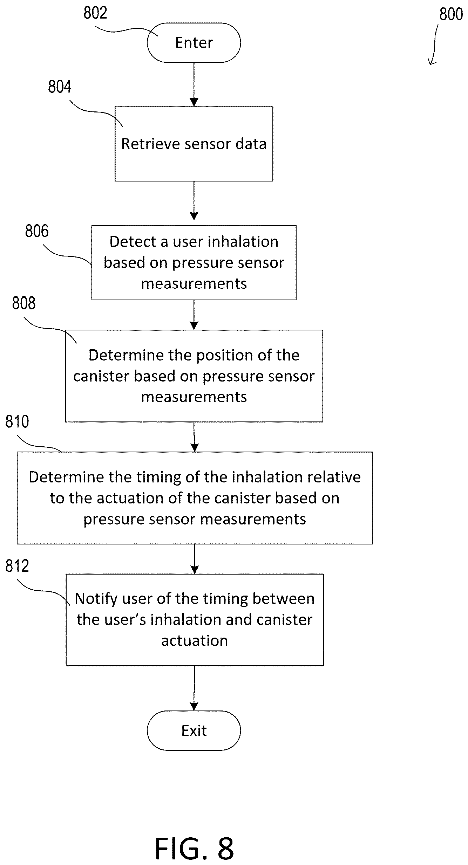

Fig. 8 is a flowchart of an example procedure that is performed by an inhaler, such as the inhaler 100 and/or the inhaler 210. The procedure 800 may be performed by the use determination system 12 and/or transmission module 14 of the inhaler 100, and/or by the electronics module 40 of the inhaler 210. In some examples, the procedure 800 may be executed in response to the use determination system 12 and/or the electronic module 40 detecting a usage event and/or waking up from a low power state. Further, although described with reference to pressure measurements received from a pressure sensor, the procedure 800 may be similarly performed in response to receiving acoustic measurements from an acoustic sensor or flow measurements from a flow sensor.

The procedure 800 may begin at 802. At 804, the inhaler may receive sensor data. As noted above, the inhaler may include a pressure sensor, and in some instances, one or more additional sensors. As such, the inhaler may receive pressure measurements at 804. In some examples, the inhaler may receive other sensor data. The other sensor data may be used to trigger the inhaler to change from a low power state (*e.g.,* an off or sleep state) to an on state, and/or cause the pressure sensor to turn on and configure the inhaler to begin receiving pressure measurements. Further, in some examples, the other sensor data may include a duration that the inhaler is shaken, the orientation of the inhaler, a temperature of the ambient air around the inhaler, a geographical location of the inhaler, *etc.*

At 806, the inhaler may detect that a user is inhaling through the inhaler based on the received sensor data (*e.g.*, pressure measurements). For example, the inhaler may compare the received pressure measurements to one or more thresholds to determine whether the user is inhaling. Alternatively or additionally, the inhaler may calculate one or more flow rates using the pressure measurements, and compare the flow rates to one or more thresholds to determine whether the user is inhaling through the inhaler. In some examples, the thresholds may be an absolute value (*e.g*., 0.1 kPA or 10 LMP) or a slope threshold (*e.g.,* a rate of change in the received pressure measurements or flow rate). Further, in some examples, the inhaler may be configured to generate a notification once the pressure measurements exceed a threshold that indicates that the user should actuate the canister (*e.g.,* to deliver the medication to a targeted region of the user's respiratory track).

At 808, the inhaler may determine the position of a medication canister of the inhaler based on the received sensor data (*e.g.,* the pressure measurements). For example, the inhaler may determine whether the canister is in an actuated position or a non-actuated position based on the pressure measurements. The inhaler may determine that the medication canister changed positions based on the received sensor data (*e.g.,* the pressure measurements). As noted herein, the inhaler may determine that the pressure measurements and/or calculated flow rates exceed a threshold or a slope, and in response, determine that the canister has moved from a first position to a second position. It should be appreciated that the procedure 800 is not described in any temporally sequential manner, and that 806 and 808 may be performed in any order or simultaneously.

At 810, the inhaler may determine the timing of the user's inhalation relative to the actuation of the canister based on the received sensor data (*e.g.,* the pressure measurements). For example, the inhaler may determine that the canister was actuated before the user inhaled, and in response, generate a late inhalation error event. The inhaler may determine that the user inhaled too far in advance of the canister being actuated (*e.g.*, based on the user's inhalation reaching too high of a value (*e.g.,* PIF) and/or occurring for too long a time period before the canister was actuated), and in response, generate a late actuation error event. Or the inhaler may generate an on-time inhalation event if the user's inhalation occurred within a predetermined time period before the canister was actuated, but did not trigger a late actuation error event.

At 812, the inhaler may notify the user of the timing between the user's inhalation and the actuation of the canister, before the procedure 800 exits. For example, the inhaler may notify the user of the occurrence of a late inhalation error event, a late actuation error event, and/or an on-time inhalation event using an indicator of the inhaler. For instance, the inhaler may play a distinctive sound through a speaker of the inhaler to indicate one or more of the events, illuminate a light on the inhaler to a distinctive color to indicate one or more of the events, etc. Alternatively or additionally, the inhaler may notify the user of the event by sending data to an external device (*e.g.,* the external device 60) that causes the external device to display a notification to the user of the event. For example, the inhaler may send any combination of the sensor data (*e.g.,* the pressure measurements), the late inhalation error event, the late actuation error event, and/or the on-time inhalation event, potentially along with one or more inhalation parameters to the external device. The external device may receive the sensor data, event(s), and/or parameters, calculate the events, if not already performed, and may display, via a display device of the external device, a notification to the user that indicates the event type and/or the associated parameters of their inhalation. For instance, in some examples, the external device may perform any combination of 808, 810, and 812.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

## Claims

1. An inhaler (210) comprising:
a housing (20) that comprises a mouthpiece (24), a medication canister (11), and an airflow channel formed between an air inlet and the mouthpiece (24);
a pressure sensor (48) configured to measure a pressure indicative of the pressure in the airflow channel, wherein the pressure sensor (48) is configured to be in a first position relative to the airflow channel when the medication canister (11) is not actuated, and in a second position relative to the airflow channel when the medication canister (11) is in an actuated position; and
a processor (42) that is configured to:
receive pressure measurements from the pressure sensor (48); and
determine that the medication canister (11) was actuated based on the pressure measurements.

2. The inhaler of claim 1, wherein the inhaler (210) is configured such that for a predetermined airflow in the airflow channel, the pressure sensor (48) is capable of measuring in the first position a first pressure and in the second position a distinguishable second pressure.

3. The inhaler of claim 1 or 2, wherein the processor (42) is configured to determine the position of the pressure sensor (48) or the medication canister (11) based on the pressure measurements received from the pressure sensor (48) while a user is inhaling through the mouthpiece (24) of the inhaler (210).

4. The inhaler of any preceding claim, wherein:
the processor (42) is configured to generate an error event based on a slope of the received pressure measurements not exceeding a slope threshold that is indicative of the canister actuation during an inhalation; and send, via a transmitter, the error event to an external device (60) for display via a user interface of the external device (60); and/or
the processor (42) is configured to generate a late inhalation error event based on the determination that the actuation of the medication canister (11) did not occur during an inhalation; and send, via a transmitter, the late inhalation error event to an external device (60) for display via a user interface of the external device (60); and/or
the processor (42) is configured to generate a good inhalation event based on a slope of the received pressure measurements exceeding a slope threshold that is indicative of the canister actuation during an inhalation; and send, via a transmitter, the good inhalation event to an external device (60) for display via a user interface of the external device (60).

5. The inhaler of any preceding claim, wherein:
the pressure sensor (48) is connected to the medication canister (11); and/or
the pressure sensor (48) comprises a differential pressure sensor or a barometric pressure sensor.

6. The inhaler of any preceding claim, wherein the processor (42) is configured to determine an inhalation event based on the pressure measurements received from the pressure sensor (48) exceeding an inhalation threshold, and determine a canister actuation event based on the pressure measurements exceeding a canister actuation threshold.

7. The inhaler of claim 6, wherein:
the canister actuation threshold is a slope threshold; and/or
the processor (42) is configured to determine that the canister (11) moved back to the first position upon the release of the canister (11) based on a slope in the pressure measurements exceeding a canister released threshold; and/or
the inhaler (210) further comprises a transmitter configured to send an indication of the inhalation and the canister actuation event to an external device (60); and/or
the inhaler (210) further comprises a receiver configured to receive the inhalation threshold or the canister actuation threshold from an external device (60); and/or
the processor (42) is configured to determine a duration of an inhalation event based on the pressure measurements received from the pressure sensor (48), optionally wherein the duration of the inhalation event starts at the time of the canister actuation event and ends when the parameters return to a predetermined value.

8. The inhaler of any preceding claim, wherein:
The processor (42) is configured to determine a peak inspiratory flow, hereinafter PIF, based on the received pressure measurements, and generate an late actuation error event based on the PIF exceeding a threshold prior to the determination that the canister (11) is actuated; and/or
the processor (42) is configured to generate an late actuation error event based on a slope of the pressure measurements exceeding a threshold for a period of time that exceeds a duration threshold prior to the determination that the canister (11) is actuated; and/or
the processor (42) and pressure sensor (48) are calibrated based on only one of the first position or the second position.

9. The inhaler of any preceding claim, wherein:
the pressure sensor (48) comprises a pressure port (49), and wherein the pressure sensor (48) is configured such that it does not extend into the inhaler housing (20) when the pressure sensor (48) is in the first position or the second position; and/or
the pressure sensor (48) comprises a pressure port (49), and wherein a gap exists between the pressure sensor (48) and the flow channel of the inhaler when the pressure sensor (48) is in the first position or is in the second position; and/or
the pressure sensor (48) comprises a pressure port (49) that is aligned with an opening between the medication canister (11) and the housing (20) when the pressure sensor (48) is connected to the medication canister (11).

10. The inhaler of any preceding claim, wherein:
the inhaler further comprises a capillary tube (650) connected to a sensor port of the pressure sensor (48), wherein the capillary tube (650) is configured to extend from the pressure sensor (48) into the housing (20); and/or
the medication canister (11) is configured to be in a first position when the medication canister (11) is not actuated, and be in a second position when the medication canister (11) is actuated to dispense medication; and/or
the processor (42) is configured with a first flow rate calibration curve for when the pressure sensor (48) is in the first position, and a second flow rate calibration curve for when the pressure sensor (48) is in the second position; and/or
the medication canister (11) is configured move from a first position relative to the housing (20) to a second position relative to the housing (20) to dispense medication.

11. The inhaler of any preceding claim, wherein:
the housing (20) comprises a hollow socket (30) configured to receive a valve stem (15) of the medication canister (11), and wherein the pressure sensor (48) is configured move relative to the hollow socket (30) in response to the medication canister (11) moving to dispense medication; and/or
the processor (42) is configured to determine a shake event of the inhaler (210) based on the pressure measurements received from the pressure sensor (48); and/or
the processor (42) is configured to determine a start time of the user's inhalation based on the pressure measurements, determine an end time of the user's inhalation based on a determination that an exhalation occurred using the pressure measurements, and determine a duration of the user's inhalation based on the start time and the end time; wherein the processor (42) is configured to send data indicating the duration of the user's inhalation to an external device (60) for display to the user.

12. A system comprising:
the inhaler (210) of claim 1 or 2, wherein the inhaler (210) is configured to send an indication to an external device (60) that actuation of the medication canister (11) occurred during inhalation based on the pressure measurements;
a computer-readable storage medium comprising executable instructions that, when executed by a processor of the external device (60), cause the processor of the external device (60) to generate a good inhalation event notification, via a user interface, that indicates that actuation of the medication canister (11) occurred during the inhalation.

13. A system comprising:
the inhaler (210) of claim 1 or 2, wherein the inhaler (210) is configured to send an indication to an external device (60) that actuation of the medication canister (11) did not occur during the inhalation; and
a computer-readable storage medium comprising executable instructions that, when executed by a processor of the external device (60), cause the processor of the external device (60) to generate an error event notification, via a user interface, that indicates that actuation of the medication canister (11) did not occur during the inhalation.

14. A system comprising:
an inhaler (210) comprising:
a housing (20) that comprises a mouthpiece (24), a medication canister (11), and an airflow channel formed between an air inlet and the mouthpiece (24);
a pressure sensor (48) configured to measure a pressure indicative of the pressure in the airflow channel, wherein the pressure sensor (48) is configured to be in a first position relative to the airflow channel when the medication canister (11) is not actuated, and in a second position relative to the airflow channel when the medication canister (11) is in an actuated position;
wherein the inhaler (210) is configured such that for a predetermined airflow in the airflow channel, the pressure sensor (48) is capable of measuring in the first position a first pressure and in the second position a distinguishable second pressure;
a wireless transmitter (46); and
a processor (42) that is configured to receive pressure measurements from the pressure sensor (48), and to send the pressure measurements to an external device (60);
a computer-readable storage medium comprising executable instructions that, when executed by a processor of the external device (69), cause the processor of the external device (60) to receive the pressure measurements, and determine whether the medication canister (11) was actuated during an inhalation based on the pressure measurements.

15. An accessory for use with an inhaler that comprises a housing and a medication canister, the accessory comprising:
a housing configured to be connected to the medication canister of the inhaler;
a pressure sensor configured to measure a pressure indicative of the pressure in an airflow channel of the inhaler, wherein the pressure sensor is configured to be in a first position relative to the airflow channel when the medication canister is not actuated, and in a second position relative to the airflow channel when the medication canister is in an actuated position; and
a processor that is configured to:
receive pressure measurements from the pressure sensor; and
determine an actuation of the medication canister based on the pressure measurements.

## Patentansprüche

1. Inhalator (210), umfassend:
ein Gehäuse (20), das ein Mundstück (24), einen Medikamentenbehälter (11) und einen Luftströmungskanal, der zwischen einem Lufteinlass und dem Mundstück (24) ausgebildet ist, umfasst;
einen Drucksensor (48), der dazu konfiguriert ist, einen Druck zu messen, der den Druck in dem Luftströmungskanal angibt, wobei der Drucksensor (48) dazu konfiguriert ist, sich in einer ersten Position relativ zu dem Luftströmungskanal zu befinden, wenn der Medikamentenbehälter (11) nicht betätigt wird, und sich in einer zweiten Position relativ zu dem Luftströmungskanal zu befinden, wenn sich der Medikamentenbehälter (11) in einer betätigten Position befindet; und
einen Prozessor (42), der zu Folgendem konfiguriert ist:
Empfangen von Druckmessungen von dem Drucksensor (48); und
Bestimmen, dass der Medikamentenbehälter (11) betätigt wurde, basierend auf den Druckmessungen.

2. Inhalator nach Anspruch 1, wobei der Inhalator (210) derart konfiguriert ist, dass für eine vorbestimmte Luftströmung in dem Luftströmungskanal der Drucksensor (48) in der Lage ist, in der ersten Position einen ersten Druck und in der zweiten Position einen unterscheidbaren zweiten Druck zu messen.

3. Inhalator nach Anspruch 1 oder 2, wobei der Prozessor (42) dazu konfiguriert ist, die Position des Drucksensors (48) oder des Medikamentenbehälters (11) basierend auf den Druckmessungen zu bestimmen, die von dem Drucksensor (48) empfangen werden, während ein Benutzer durch das Mundstück (24) des Inhalators (210) inhaliert.

4. Inhalator nach einem vorhergehenden Anspruch, wobei:
der Prozessor (42) dazu konfiguriert ist, ein Fehlerereignis basierend darauf zu erzeugen, dass eine Steigung der empfangenen Druckmessungen einen Steigungsschwellenwert, der eine Behälterbetätigung angibt, während einer Inhalation nicht überschreitet; und über einen Sender das Fehlerereignis an eine externe Vorrichtung (60) zur Anzeige über eine Benutzerschnittstelle der externen Vorrichtung (60) zu senden; und/oder
der Prozessor (42) dazu konfiguriert ist, ein Spätinhalationsfehlerereignis basierend auf der Bestimmung zu erzeugen, dass die Betätigung des Medikamentenbehälters (11) während einer Inhalation nicht aufgetreten ist; und über einen Sender das Spätinhalationsfehlerereignis an eine externe Vorrichtung (60) zur Anzeige über eine Benutzerschnittstelle der externen Vorrichtung (60) zu senden; und/oder
der Prozessor (42) dazu konfiguriert ist, ein Ereignis einer guten Inhalation basierend darauf zu erzeugen, dass eine Steigung der empfangenen Druckmessungen einen Steigungsschwellenwert,
der die Behälterbetätigung angibt, während einer Inhalation überschreitet; und über einen Sender das Ereignis einer guten Inhalation an eine externe Vorrichtung (60) zur Anzeige über eine Benutzerschnittstelle der externen Vorrichtung (60) zu senden.

5. Inhalator nach einem vorhergehenden Anspruch, wobei:
der Drucksensor (48) mit dem Medikamentenbehälter (11) verbunden ist; und/oder
der Drucksensor (48) einen Differenzdrucksensor oder einen barometrischen Drucksensor umfasst.

6. Inhalator nach einem vorhergehenden Anspruch, wobei der Prozessor (42) dazu konfiguriert ist, ein Inhalationsereignis basierend darauf zu bestimmen, dass die von dem Drucksensor (48) empfangenen Druckmessungen einen Inhalationsschwellenwert überschreiten, und ein Behälterbetätigungsereignis basierend darauf zu bestimmen, dass die Druckmessungen einen Behälterbetätigungsschwellenwert überschreiten.

7. Inhalator nach Anspruch 6, wobei:
der Behälterbetätigungsschwellenwert ein Steigungsschwellenwert ist; und/oder
der Prozessor (42) dazu konfiguriert ist, basierend darauf, dass eine Steigung in den Druckmessungen einen Behälterfreigabeschwellenwert überschreitet, zu bestimmen, dass sich der Behälter (11) nach dem Freigeben des Behälters (11) zurück in die erste Position bewegt hat; und/oder
der Inhalator (210) ferner einen Sender umfasst, der dazu konfiguriert ist, eine Angabe des Inhalations- und des Behälterbetätigungsereignisses an eine externe Vorrichtung (60) zu senden; und/oder
der Inhalator (210) ferner einen Empfänger umfasst, der dazu konfiguriert ist, den Inhalationsschwellenwert oder den Behälterbetätigungsschwellenwert von einer externen Vorrichtung (60) zu empfangen; und/oder
der Prozessor (42) dazu konfiguriert ist, eine Dauer eines Inhalationsereignisses basierend auf den von dem Drucksensor (48) empfangenen Druckmessungen zu bestimmen, wobei optional die Dauer des Inhalationsereignisses zu dem Zeitpunkt des Behälterbetätigungsereignisses beginnt und endet, wenn die Parameter zu einem vorbestimmten Wert zurückkehren.

8. Inhalator nach einem vorhergehenden Anspruch, wobei:
der Prozessor (42) dazu konfiguriert ist, einen maximalen inspiratorischen Atemstrom, nachstehend PIF, basierend auf den empfangenen Druckmessungen zu bestimmen und ein Spätbetätigungsfehlerereignis basierend darauf zu erzeugen, dass der PIF vor der Bestimmung,
dass der Behälter (11) betätigt wird, einen Schwellenwert überschreitet; und/oder der Prozessor (42) dazu konfiguriert ist, ein Spätbetätigungsfehlerereignis basierend darauf zu erzeugen, dass eine Steigung der Druckmessungen einen Schwellenwert für einen Zeitraum, der einen Dauerschwellenwert überschreitet, vor der Bestimmung, dass der Behälter (11) betätigt wird, überschreitet; und/oder
der Prozessor (42) und der Drucksensor (48) basierend auf nur einer von der ersten Position oder der zweiten Position kalibriert werden.

9. Inhalator nach einem vorhergehenden Anspruch, wobei:
der Drucksensor (48) einen Druckanschluss (49) umfasst und wobei der Drucksensor (48) derart konfiguriert ist, dass er sich nicht in das Inhalatorgehäuse (20) erstreckt, wenn sich der Drucksensor (48) in der ersten Position oder der zweiten Position befindet; und/oder
der Drucksensor (48) einen Druckanschluss (49) umfasst und wobei ein Spalt zwischen dem Drucksensor (48) und dem Strömungskanal des Inhalators besteht, wenn sich der Drucksensor (48) in der ersten Position befindet oder sich in der zweiten Position befindet; und/oder
der Drucksensor (48) einen Druckanschluss (49) umfasst, der mit einer Öffnung zwischen dem Medikamentenbehälter (11) und dem Gehäuse (20) ausgerichtet ist, wenn der Drucksensor (48) mit dem Medikamentenbehälter (11) verbunden ist.

10. Inhalator nach einem vorhergehenden Anspruch, wobei:
der Inhalator ferner ein Kapillarrohr (650) umfasst, das mit einem Sensoranschluss des Drucksensors (48) verbunden ist, wobei das Kapillarrohr (650) dazu konfiguriert ist, sich von dem Drucksensor (48) in das Gehäuse (20) zu erstrecken; und/oder
der Medikamentenbehälter (11) dazu konfiguriert ist, sich in einer ersten Position zu befinden, wenn der Medikamentenbehälter (11) nicht betätigt wird, und sich in einer zweiten Position zu befinden, wenn der Medikamentenbehälter (11) betätigt wird, um Medikamente abzugeben; und/oder
der Prozessor (42) mit einer ersten Strömungsratenkalibrierungskurve für den Fall, dass sich der Drucksensor (48) in der ersten Position befindet, und einer zweiten Strömungsratenkalibrierungskurve für den Fall, dass sich der Drucksensor (48) in der zweiten Position befindet, konfiguriert ist; und/oder
der Medikamentenbehälter (11) dazu konfiguriert ist, sich von einer ersten Position relativ zu dem Gehäuse (20) zu einer zweiten Position relativ zu dem Gehäuse (20) zu bewegen, um Medikamente abzugeben.

11. Inhalator nach einem vorhergehenden Anspruch, wobei:
das Gehäuse (20) einen hohlen Sockel (30) umfasst, der dazu konfiguriert ist, einen Ventilschaft (15) des Medikamentenbehälters (11) aufzunehmen, und wobei der Drucksensor (48) dazu konfiguriert ist, sich als Reaktion darauf, dass sich der Medikamentenbehälter (11) bewegt, um Medikamente abzugeben, relativ zu dem hohlen Sockel (30) zu bewegen; und/oder
der Prozessor (42) dazu konfiguriert ist, ein Schüttelereignis des Inhalators (210) basierend auf den von dem Drucksensor (48) empfangenen Druckmessungen zu bestimmen; und/oder
der Prozessor (42) dazu konfiguriert ist, eine Startzeit der Inhalation des Benutzers basierend auf den Druckmessungen zu bestimmen, eine Endzeit der Inhalation des Benutzers basierend auf einer Bestimmung, dass eine Ausatmung stattgefunden hat, unter Verwendung der Druckmessungen zu bestimmen und eine Dauer der Inhalation des Benutzers basierend auf der Startzeit und der Endzeit zu bestimmen; wobei der Prozessor (42) dazu konfiguriert ist, Daten, die die Dauer der Inhalation des Benutzers angeben, an eine externe Vorrichtung (60) zur Anzeige für den Benutzer zu senden.

12. System, umfassend:
den Inhalator (210) nach Anspruch 1 oder 2, wobei der Inhalator (210) dazu konfiguriert ist, eine Angabe an eine externe Vorrichtung (60) zu senden, dass eine Betätigung des Medikamentenbehälters (11) basierend auf den Druckmessungen während Inhalation aufgetreten ist;
ein computerlesbares Speichermedium, das ausführbare Anweisungen umfasst, die, wenn sie durch einen Prozessor der externen Vorrichtung (60) ausgeführt werden, den Prozessor der externen Vorrichtung (60) dazu veranlassen, über eine Benutzerschnittstelle eine Benachrichtigung über ein Ereignis einer guten Inhalation zu erzeugen, die angibt, dass eine Betätigung des Medikamentenbehälters (11) während der Inhalation aufgetreten ist.

13. System, umfassend:
den Inhalator (210) nach Anspruch 1 oder 2, wobei der Inhalator (210) dazu konfiguriert ist, eine Angabe an eine externe Vorrichtung (60) zu senden, dass eine Betätigung des Medikamentenbehälters (11) während der Inhalation nicht aufgetreten ist; und
ein computerlesbares Speichermedium, das ausführbare Anweisungen umfasst, die, wenn sie durch einen Prozessor der externen Vorrichtung (60) ausgeführt werden, den Prozessor der externen Vorrichtung (60) dazu veranlassen, über eine Benutzerschnittstelle eine Benachrichtigung über ein Fehlerereignis zu erzeugen, die angibt, dass eine Betätigung des Medikamentenbehälters (11) während der Inhalation nicht aufgetreten ist.

14. System, umfassend:
einen Inhalator (210), der Folgendes umfasst:
ein Gehäuse (20), das ein Mundstück (24), einen Medikamentenbehälter (11) und einen Luftströmungskanal, der zwischen einem Lufteinlass und dem Mundstück (24) ausgebildet ist, umfasst;
einen Drucksensor (48), der dazu konfiguriert ist, einen Druck zu messen, der den Druck in dem Luftströmungskanal angibt, wobei der Drucksensor (48) dazu konfiguriert ist, sich in einer ersten Position relativ zu dem Luftströmungskanal zu befinden, wenn der Medikamentenbehälter (11) nicht betätigt wird, und sich in einer zweiten Position relativ zu dem Luftströmungskanal zu befinden, wenn sich der Medikamentenbehälter (11) in einer betätigten Position befindet;
wobei der Inhalator (210) derart konfiguriert ist, dass für eine vorbestimmte Luftströmung in dem Luftströmungskanal der Drucksensor (48) in der Lage ist, in der ersten Position einen ersten Druck und in der zweiten Position einen unterscheidbaren zweiten Druck zu messen;
einen drahtlosen Sender (46); und
einen Prozessor (42), der dazu konfiguriert ist, Druckmessungen von dem Drucksensor (48) zu empfangen und die Druckmessungen an eine externe Vorrichtung (60) zu senden;
ein computerlesbares Speichermedium, das ausführbare Anweisungen umfasst, die, wenn sie durch einen Prozessor der externen Vorrichtung (69) ausgeführt werden, den Prozessor der externen Vorrichtung (60) dazu veranlassen, die Druckmessungen zu empfangen, und basierend auf den Druckmessungen zu bestimmen, ob der Medikamentenbehälter (11) während einer Inhalation betätigt wurde.

15. Zubehör zur Verwendung mit einem Inhalator, der ein Gehäuse und einen Medikamentenbehälter umfasst, wobei das Zubehör Folgendes umfasst:
ein Gehäuse, das dazu konfiguriert ist, mit dem Medikamentenbehälter des Inhalators verbunden zu werden;
einen Drucksensor, der dazu konfiguriert ist, einen Druck zu messen, der den Druck in einem Luftströmungskanal des Inhalators angibt, wobei der Drucksensor dazu konfiguriert ist, sich in einer ersten Position relativ zu dem Luftströmungskanal zu befinden, wenn der Medikamentenbehälter nicht betätigt wird, und sich in einer zweiten Position relativ zu dem Luftströmungskanal zu befinden, wenn sich der Medikamentenbehälter in einer betätigten Position befindet; und
einen Prozessor, der zu Folgendem konfiguriert ist:
Empfangen von Druckmessungen von dem Drucksensor; und
Bestimmen einer Betätigung des Medikamentenbehälters basierend auf den Druckmessungen.

## Revendications

1. Inhalateur (210) comprenant :
un boîtier (20) qui comprend un embout buccal (24), une cartouche de médicament (11) et un canal d'écoulement d'air formé entre une entrée d'air et l'embout buccal (24) ;
un capteur de pression (48) configuré pour mesurer une pression indiquant la pression dans le canal d'écoulement d'air, ledit capteur de pression (48) étant configuré pour se trouver dans une première position par rapport au canal d'écoulement d'air lorsque la cartouche de médicament (11) n'est pas actionnée, et dans une seconde position par rapport au canal d'écoulement d'air lorsque la cartouche de médicament (11) se trouve dans une position actionnée ; et
un processeur (42) qui est configuré pour :
recevoir des mesures de pression en provenance du capteur de pression (48) ; et
déterminer que la cartouche de médicament (11) a été actionnée sur la base des mesures de pression.

2. Inhalateur selon la revendication 1, ledit inhalateur (210) étant configuré de sorte que pour un écoulement d'air prédéterminé dans le canal d'écoulement d'air, le capteur de pression (48) soit capable de mesurer dans la première position une première pression et dans la seconde position une seconde pression pouvant être différenciée.

3. Inhalateur selon la revendication 1 ou 2, ledit processeur (42) étant configuré pour déterminer la position du capteur de pression (48) ou de la cartouche de médicament (11) sur la base des mesures de pression reçues du capteur de pression (48) pendant qu'un utilisateur inspire par l'embout buccal (24) de l'inhalateur (210).

4. Inhalateur selon l'une quelconque des revendications précédentes :
ledit processeur (42) étant configuré pour générer un événement d'erreur sur la base d'une pente des mesures de pression reçues ne dépassant pas un seuil de pente qui indiquant l'actionnement de la cartouche pendant une inhalation ; et envoyer, par l'intermédiaire d'un émetteur, l'événement d'erreur à un dispositif externe (60) pour l'affichage par l'intermédiaire d'une interface utilisateur du dispositif externe (60) ; et/ou
ledit processeur (42) étant configuré pour générer un événement d'erreur d'inhalation tardive sur la base de la détermination que l'actionnement de la cartouche de médicament (11) ne s'est pas produit pendant une inhalation ; et envoyer, par l'intermédiaire d'un émetteur, l'événement d'erreur d'inhalation tardive à un dispositif externe (60) pour l'affichage par l'intermédiaire d'une interface utilisateur du dispositif externe (60) ; et/ou
ledit processeur (42) étant configuré pour générer un bon événement d'inhalation sur la base d'une pente des mesures de pression reçues dépassant un seuil de pente qui indique l'actionnement de la cartouche pendant une inhalation ; et envoyer, par l'intermédiaire d'un émetteur, le bon événement d'inhalation à un dispositif externe (60) pour l'affichage par l'intermédiaire d'une interface utilisateur du dispositif externe (60).

5. Inhalateur selon l'une quelconque des revendications précédentes :
ledit capteur de pression (48) étant raccordé à la cartouche de médicament (11) ; et/ou
ledit capteur de pression (48) comprenant un capteur de pression différentielle ou un capteur de pression barométrique.

6. Inhalateur selon l'une quelconque des revendications précédentes, ledit processeur (42) étant configuré pour déterminer un événement d'inhalation sur la base des mesures de pression reçues du capteur de pression (48) dépassant un seuil d'inhalation, et déterminer un événement d'actionnement de cartouche sur la base des mesures de pression dépassant un seuil d'actionnement de cartouche.

7. Inhalateur selon la revendication 6 :
ledit seuil d'actionnement de la cartouche étant un seuil de pente ; et/ou
ledit processeur (42) étant configuré pour déterminer que la cartouche (11) est revenue dans la première position lors de la libération de la cartouche (11) sur la base d'une pente des mesures de pression dépassant un seuil de libération de cartouche ; et/ou
ledit inhalateur (210) comprenant en outre un émetteur configuré pour envoyer une indication de l'inhalation et de l'événement d'actionnement de la cartouche à un dispositif externe (60) ; et/ou
ledit inhalateur (210) comprenant en outre un récepteur configuré pour recevoir le seuil d'inhalation ou le seuil d'actionnement de la cartouche en provenance d'un dispositif externe (60) ; et/ou
ledit processeur (42) étant configuré pour déterminer la durée d'un événement d'inhalation sur la base des mesures de pression reçues du capteur de pression (48), éventuellement ladite durée de l'événement d'inhalation commençant au moment de l'événement d'actionnement de la cartouche et se terminant lorsque les paramètres reviennent à une valeur prédéfinie.

8. Inhalateur selon l'une quelconque des revendications précédentes :
ledit processeur (42) étant configuré pour déterminer un débit inspiratoire de pointe, ci-après PIF, sur la base des mesures de pression reçues, et générer un événement d'erreur d'actionnement tardif sur la base du PIF dépassant un seuil avant la détermination que la cartouche (11) est actionnée ; et/ou
ledit processeur (42) étant configuré pour générer un événement d'erreur d'actionnement tardif sur la base d'une pente des mesures de pression dépassant un seuil pendant une période de temps qui dépasse un seuil de durée avant la détermination de l'actionnement de la cartouche (11) ; et/ou
ledit processeur (42) et ledit capteur de pression (48) étant étalonnés sur la seule base de la première position ou de la seconde position.

9. Inhalateur selon l'une quelconque des revendications précédentes :
ledit capteur de pression (48) comprenant un orifice de pression (49), et ledit capteur de pression (48) étant configuré de sorte qu'il ne s'étende pas dans le boîtier d'inhalateur (20) lorsque le capteur de pression (48) se trouve dans la première position ou la seconde position ; et/ou
ledit capteur de pression (48) comprenant un orifice de pression (49), et un espace existant entre le capteur de pression (48) et le canal d'écoulement de l'inhalateur lorsque le capteur de pression (48) se trouve dans la première position ou se trouve dans la seconde position ; et/ou
ledit capteur de pression (48) comprenant un orifice de pression (49) qui est aligné avec une ouverture entre la cartouche de médicament (11) et le boîtier (20) lorsque ledit capteur de pression (48) est raccordé à la cartouche de médicament (11).

10. Inhalateur selon l'une quelconque des revendications précédentes :
ledit inhalateur comprenant en outre un tube capillaire (650) raccordé à un orifice de capteur du capteur de pression (48), ledit tube capillaire (650) étant configuré pour s'étendre depuis le capteur de pression (48) dans le boîtier (20) ; et/ou
ladite cartouche de médicament (11) étant configurée pour se trouver dans une première position lorsque la cartouche de médicament (11) n'est pas actionnée, et se trouver dans une seconde position lorsque la cartouche de médicament (11) est actionnée pour distribuer le médicament ; et/ou
ledit processeur (42) étant configuré avec une première courbe d'étalonnage de débit lorsque le capteur de pression (48) se trouve dans la première position, et une seconde courbe d'étalonnage de débit lorsque le capteur de pression (48) se trouve dans la seconde position ; et/ou
ladite cartouche de médicament (11) étant configurée pour se déplacer d'une première position par rapport au boîtier (20) à une seconde position par rapport au boîtier (20) pour distribuer le médicament.

11. Inhalateur selon l'une quelconque des revendications précédentes :
ledit boîtier (20) comprenant une douille creuse (30) configurée pour recevoir une tige de valve (15) de la cartouche de médicament (11), et ledit capteur de pression (48) étant configuré pour se déplacer par rapport à la douille creuse (30) en réponse au déplacement de la cartouche de médicament (11) pour distribuer le médicament ; et/ou
ledit processeur (42) étant configuré pour déterminer un événement d'agitation de l'inhalateur (210) sur la base des mesures de pression reçues du capteur de pression (48) ; et/ou
ledit processeur (42) étant configuré pour déterminer l'instant de début de l'inhalation de l'utilisateur sur la base des mesures de pression, déterminer l'instant de fin de l'inhalation de l'utilisateur sur la base de la détermination qu'une expiration s'est produite à l'aide des mesures de pression, et déterminer la durée de l'inhalation de l'utilisateur sur la base de l'instant de début et de l'instant de fin ; ledit processeur (42) étant configuré pour envoyer des données indiquant la durée de l'inhalation de l'utilisateur à un dispositif externe (60) pour l'affichage à l'utilisateur.

12. Système comprenant :
ledit inhalateur (210) selon la revendication 1 ou 2, ledit inhalateur (210) étant configuré pour envoyer l'indication à un dispositif externe (60) que l'actionnement de la cartouche de médicament (11) s'est produit pendant l'inhalation sur la base des mesures de pression ;
un support de stockage lisible par ordinateur comprenant des instructions exécutables qui, lorsqu'elles sont exécutées par un processeur du dispositif externe (60), amènent le processeur du dispositif externe (60) à générer une bonne notification d'événement d'inhalation, par l'intermédiaire d'une interface utilisateur, qui indique que l'actionnement de la cartouche de médicament (11) s'est produit pendant l'inhalation.

13. Système comprenant :
ledit inhalateur (210) selon la revendication 1 ou 2, ledit inhalateur (210) étant configuré pour envoyer l'indication à un dispositif externe (60) que l'actionnement de la cartouche de médicament (11) ne s'est pas produit pendant l'inhalation ; et
un support de stockage lisible par ordinateur comprenant des instructions exécutables qui, lorsqu'elles sont exécutées par un processeur du dispositif externe (60), amènent le processeur du dispositif externe (60) à générer une notification d'événement d'erreur, par l'intermédiaire d'une interface utilisateur, qui indique que l'actionnement de la cartouche de médicament (11) ne s'est pas produit pendant l'inhalation.

14. Système comprenant :
un inhalateur (210) comprenant :
un boîtier (20) qui comprend un embout buccal (24), une cartouche de médicament (11) et un canal d'écoulement d'air formé entre une entrée d'air et l'embout buccal (24) ;
un capteur de pression (48) configuré pour mesurer une pression indiquant la pression dans le canal d'écoulement d'air, ledit capteur de pression (48) étant configuré pour se trouver dans une première position par rapport au canal d'écoulement d'air lorsque la cartouche de médicament (11) n'est pas actionnée, et dans une seconde position par rapport au canal d'écoulement d'air lorsque la cartouche de médicament (11) se trouve dans une position actionnée ;
ledit inhalateur (210) étant configuré de sorte que pour un écoulement d'air prédéfini dans le canal d'écoulement d'air, le capteur de pression (48) soit capable de mesurer dans la première position une première pression et dans la seconde position une seconde pression pouvant être différenciée ;
un émetteur sans fil (46) ; et
un processeur (42) qui est configuré pour recevoir des mesures de pression du capteur de pression (48) et pour envoyer les mesures de pression à un dispositif externe (60) ;
un support de stockage lisible par ordinateur comprenant des instructions exécutables qui, lorsqu'elles sont exécutées par un processeur du dispositif externe (69), amènent le processeur du dispositif externe (60) à recevoir les mesures de pression et déterminer si la cartouche de médicament (11) a été actionnée pendant une inhalation sur la base des mesures de pression.

15. Accessoire destiné à être utilisé avec un inhalateur qui comprend un boîtier et une cartouche de médicament, l'accessoire comprenant :
un boîtier configuré pour être raccordé à la cartouche de médicament de l'inhalateur ;
un capteur de pression configuré pour mesurer une pression indiquant la pression dans un canal d'écoulement d'air de l'inhalateur, ledit capteur de pression étant conçu pour se trouver dans une première position par rapport au canal d'écoulement d'air lorsque la cartouche de médicament n'est pas actionnée, et dans une seconde position par rapport au canal d'écoulement d'air lorsque la cartouche de médicament se trouve dans une position actionnée ; et
un processeur qui est configuré pour :
recevoir des mesures de pression en provenance du capteur de pression ; et
déterminer un actionnement de la cartouche de médicament sur la base des mesures de pression.
